# EUROPEAN PATENT APPLICATION

(11) **EP 4 417 614 A1**
(43) Date of publication of application: **21.08.2024**
(21) Application number: 22880381.3
(22) Date of filing: 13.10.2022
(51) Int. Cl.: C07F 5/04, C07F 5/02, A61K 31/69, A61P 17/06, A61P 25/28, A61P 35/00, A61P 37/00

(54) **BORONIC ACID DERIVATIVE**

(30) Priority: 14.10.2021 CN 202111200305; 07.02.2022 CN 202210110439; 30.08.2022 CN 202211046052
(71) Applicant: Shouyao Holdings (Beijing) Co., Ltd., Beijing 100176 (CN)
(72) Inventor: WANG, Yeliu, Beijing 100195 (CN); LU, Chang, Beijing 100195 (CN); ZHAO, Sheng, Beijing 100195 (CN); LI, Jijun, Beijing 100195 (CN); HE, Weinan, Beijing 100195 (CN); LI, Hongjuan, Beijing 100195 (CN)
(74) Representative: Wallinger, Michael
(86) International application number: PCT/CN2022/125099
(87) International publication number: WO 2023/061445

(57) **Abstract**

Disclosed is a boronic acid derivative. Provided are a compound of formula (I) or a pharmaceutically acceptable salt, solvate, polymorph or isomer thereof, a pharmaceutical composition containing the same, and the use thereof in the treatment of lmp7-related diseases.

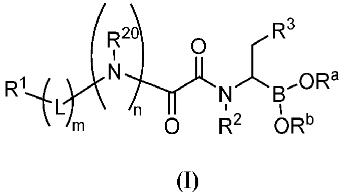

## Description

### Cross-reference

This application claims priority to Chinese Patent Application No. 202111200305.3, entitled "Boric Acid Derivative", filed on October 14, 2021, to Chinese Patent Application No. 202210110439.4, entitled "Boric Acid Derivative", filed on February 7, 2022, and to Chinese Patent Application No. 202211046052.3, entitled "Boric Acid Derivative", filed on August 30, 2022, the entire disclosures of which are incorporated herein by reference in their entirety.

### Technical Field

The present invention relates to a novel boronic acid derivative of formula (I) or pharmaceutically acceptable salt thereof. Also provided herein are pharmaceutical compositions containing such compounds and the preparation method thereof. The compounds described herein are useful in the treatment or prevention of diseases associated with the immunoproteasome.

### Background Art

The ubiquitin-proteasome system (UPS) is present in all eukaryotic cells and is responsible for intracellular misfolding or degradation of redundant proteins. By regulating protein level homeostasis, almost all important activities such as signal transduction, transcriptional regulation, cell differentiation, and apoptosis are regulated. The 26S proteasome, (the sedimentation coefficient of proteasome density gradient centrifugation is 26S, so it is known as 26S proteasome) can be structurally divided into two parts: 19S regulatory particles and 20S core particles. The 19S regulatory particles are responsible for recognizing and unfolding ubiquitin-tagged proteins, and finally delivering unfolded proteins to the 20S core particles for degradation. The 20S proteasome has a barrel structure consisting of four loops. The outer two loops, each containing seven α subunits, act on the one hand as binding sites for regulatory particles and on the other hand act as "gates" preventing the unregulated entry of proteins into the interior of the core particle. The inner two loops, each containing seven β subunits, contain protease active subunits β 1c, β 2c, and β 5c for protein hydrolysis reactions. In hematopoietic cells and cells stimulated by IFN-γ or TNF-a, these active subunits are replaced by β1i (LMP2, low molecular weight polypeptide 2), β2i (MECL-1, multicatalytic endopeptidase complex analogue-1) and β5i (LMP7) to form immunoproteasome [Michael Basler et al. EMBO reports, 2018]. LMP7, encoded by the PSMB8 gene, contains 276 amino acids and is a small protein of about 30 kDa. LMP7 is the core catalytic subunit of the immunoproteasome, with chymotrypsin activity, and plays an important role in proteolysis by the immunoproteasome [A . Arkhjami et al, Immune and non-immune functions of the immunoproteasome, Frontiers in Bioscience, 17(1):1904, 2012].

The function of the immunoproteasome in the process of immunization has been well studied, especially its antigen-presenting function. Hydrolysis of the catalytic subunit of the immunoproteasome produces a polypeptide that is presented to the cell surface by major histocompatibility complex (MHC)-1, leading to a cytotoxic T lymphocyte (CTL). Compared to the proteasome, the immunoproteasome can hydrolyze proteins and present antigens more efficiently, and the resulting antigens can cause more intense CTLs. Several studies have demonstrated that immunoproteasome can regulate cytokine production. Selective inhibition of LMP7 with small molecule inhibitors inhibits both IL-23 in monocytes and TNF-α and IL-6 in T cells, and a similar phenomenon was observed in animal models of rheumatoid arthritis [T. Muchamuel et al, A selective inhibitor of the immunoproteasome subunit LMP7 blocks cytokine production and attenuates progression of experimental arthritis, Nat Med, 15(7), 781-7, 2009]. In addition, the function of the immunoproteasome in T cell differentiation, proliferation, and apoptosis has been validated in several studies [C. M. Caudill et al, T cells lacking immunoproteasome subunits MECL-1 and LMP7 hyperproliferate in response to polyclonal mitogens, J. Immunol, 176(7), 4075-82, 2006]. In addition to immune function, the immunoproteasome plays a role in maintaining protein homeostasis in cytokine-induced oxidative stress. Oxidative stress releases free radicals, leading to the accumulation of a large amount of damaged proteins that exceed the clearance capacity of ordinary proteasomes, ultimately leading to cell death. The immunoproteasome can efficiently remove protein accumulation and maintain cellular homeostasis. In LMP7/β5i- and LMP2/β1i-deficient mice, accumulation of oxidized and polyubiquitinated proteins in the liver and brain was observed [U. Seifert et al, Immunoproteasomes preserve protein homeostasis upon interferon-induced oxidative stress, Cell, 142(4), 613- 24, 2010].

The immunoproteasome is associated with a variety of diseases. Studies have shown that immunoproteasome is highly expressed in hematological cancers and that selective inhibition of β1i and LMP7 can effectively inhibit the growth of patient-derived cells and tumor models [U. Seifert et al, Immunoproteasomes preserve protein homeostasis upon interferon-induced oxidative stress, Cell, 142(4), 613- 24, 2010]. In a study of 668 breast cancer patients, LMP7 was highly expressed in tumors in 40% of patients [M. Lee et al. Expression of Immunoproteasome Subunit LMP7 in Breast Cancer and Its Association with Immune-Related Markers, Cancer Research and Treatment, 51 (1), 2018]. The immunoproteasome promotes the development and progression of colorectal cancer, and inhibitors of LMP7 can effectively inhibit the development of colorectal cancer in mouse models [J. Koerner et al, Inhibition and deficiency of the immunoproteasome subunit LMP7 suppress the development and progression of colorectal carcinoma in mice, Oncotarget, 8(31):50873-50888, 2017]. Recent evidence suggests that the immunoproteasome is associated with autoimmune diseases and may be a hot target for the treatment of such diseases. The immunoproteasome is highly expressed in autoimmune diseases such as rheumatoid arthritis and inflammatory bowel disease [T. Egerer et al, Tissue-specific up-regulation of theproteasome subunit β5i (LMP7) in Sjogren's syndrome, Arthritis Rheum, 54(5), 1501-8, 2006]. In both mouse models of arthritis, selective inhibitors of LMP7 reduce the extent of inflammatory infiltrates and cytokine levels, alleviating the symptoms of arthritis [J. Koerner et al, Inhibition and deficiency of the immunoproteasome subunit LMP7 suppress the development and progression of colorectal carcinoma in mice, Oncotarget, 8(31):50873-50888, 2017]. It is reported that the immunoproteasome is associated with neurodegenerative diseases and is highly expressed in the brain of Alzheimer's disease [M. DiazHernandez et al, Neuronal Induction of the Immunoproteasome in Huntington's Disease, Journal of Neuroscience, 23(37):11653-11661, 2003].

LMP7, the catalytic subunit of immunoproteasome, is a new target for the treatment of many diseases and has a broad development space. Selective inhibitors of LMP7 have an absolute advantage in safety over a broad spectrum of proteasome inhibitors. WO2019099582A1 and WO2019038250A1, among others, disclose certain LMP7 inhibitors and methods of using them to treat related diseases.

This patent describes a boronic acid derivative with good inhibitory activity against LMP7 and good selectivity against other proteasomes. Furthermore, these compounds have excellent oral bioavailability, plasma protein adsorption, pharmacokinetic characteristics, and *in-vivo* activity in animals, with lower toxicity, CYP-inhibition, and a larger drug window.

### Summary of the Invention

.In one aspect, the present invention provides a compound of formula (I), or a pharmaceutically acceptable salt, solvate, polymorph, or tautomer thereof, 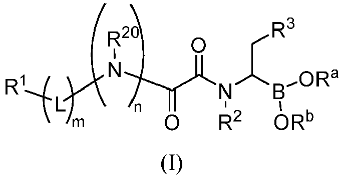 wherein,
R^{a} and R^{b} are each independently selected from H and C₁₋₆ alkyl, or R^{a} and R^{b} can join together to form a 3-10 membered heterocycle, wherein the heterocycle can be optionally substituted with (C=O), halogen, or C₁₋₆ alkyl; the alkyl can be optionally substituted with -COOH; the heterocycle can be optionally fused with a phenyl ring or a 5-6 membered heteroaryl ring; the phenyl ring or 5-6 membered heteroaryl ring can be optionally substituted with halogen or C₁₋₆ alkyl,
R² and R²⁰ are each independently selected from H and C₁₋₆ alkyl,
L is -CR⁴R⁵-,
R⁴ and R⁵ are each independently selected from H, C₁₋₆ alkyl and 3-8 membered cycloalkyl,
R¹ is selected from -OH, C₁₋₆ alkyl, -O-C₁₋₆ alkyl, C₂₋₆ alkenyl, C₂₋₆ alkynyl, 3-12 membered cycloalkyl, 3-12 membered heterocycloalkyl, 6-10 membered aryl, and 5-12 membered heteroaryl, wherein the alkyl, cycloalkyl and heterocycloalkyl can be optionally substituted with (=O); the cycloalkyl, heterocycloalkyl, aryl and heteroaryl can be optionally substituted with halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 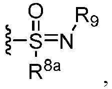 or 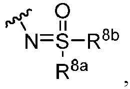 or,
R¹ and R²⁰ join together to form a 3-12 membered heterocycle, wherein the heterocycle can be optionally substituted with (=O), halogen, -CN, -OH, -NH₂, NOz, -(CH₂)₀₋₆-R⁷ -(CH₂)₀₋₆-CF₃, -O-R⁶, -NR¹⁰R⁶, C₁₋₆ alkyl, -(CH₂)₀₋₃-(CO)-R, ⁶ -(CH₂)₀₋₃-(CO)-NH-R⁶, -(CH₂)₀₋₃-NH-(CO)-R⁶, 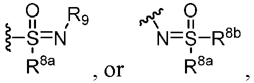
R³ is selected from C₁₋₆ alkyl, 6-10 membered aryl, and 5-12 membered heteroaryl, wherein the aryl and heteroaryl can be optionally substituted with halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, or -(CH₂)₀₋₃-NH-(CO)-R⁶,
R⁶ are each independently selected from C₁₋₆ alkyl, 3-8 membered cycloalkyl, 3-8 membered heterocycloalkyl, 6-10 membered aryl, and 5-12 membered heteroaryl, wherein the alkyl, cycloalkyl, heterocycloalkyl, aryl and heteroaryl can be optionally substituted with halogen, -CN, C₁₋₆ alkyl, -O-C₁₋₆ alkyl, 3-8 membered cycloalkyl, 3-8 membered heterocycloalkyl, 6-10 membered aryl, or 5-12 membered heteroaryl,
R¹⁰ are each independently selected from H and C₁₋₆ alkyl,
R⁷ are each independently selected from 3-8 membered cycloalkyl, 3-8 membered heterocycloalkyl, 6-10 membered aryl, and 5-12 membered heteroaryl, wherein the cycloalkyl, heterocycloalkyl, aryl and heteroaryl can be optionally substituted with halogen, -CN, -OH, -NH₂, NOz, -O-C₁₋₆ alkyl, -N(C₁₋₆ alkyl) (C₁₋₆ alkyl), or C₁₋₆ alkyl,
R^{8a} and R^{8b} are each independently selected from C₁₋₆ alkyl, 3-8 membered cycloalkyl, 3-8 membered heterocycloalkyl, 6-10 membered aryl, and 5-12 membered heteroaryl, or
R^{8a} and R^{8b} can join together to form a 3-8 membered heterocycle,
R⁹ is selected from H, C₁₋₆ alkyl, 3-8 membered cycloalkyl, 3-8 membered heterocycloalkyl, 6-10 membered aryl, and 5-12 membered heteroaryl,
m is 0, 1, 2 or 3,
n is 0 or 1.

In some embodiments, R^{a} and R^{b} are each independently selected from H and C₁₋₆ alkyl, or R^{a} and R^{b} can join together to form a 3-10 membered heterocycle,
R¹ is selected from C₁₋₆ alkyl, -O-C₁₋₆ alkyl, C₂₋₆ alkenyl, C₂₋₆ alkynyl, 3-12 membered cycloalkyl, 3-12 membered heterocycloalkyl, 6-10 membered aryl, and 5-12 membered heteroaryl, wherein the alkyl, cycloalkyl and heterocycloalkyl can be optionally substituted with (=O); the cycloalkyl, heterocycloalkyl, aryl and heteroaryl can be optionally substituted with halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 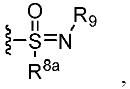 or 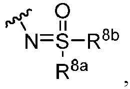 or,
R¹ and R²⁰ join together to form a 3-12 membered heterocycle, wherein the heterocycle can be optionally substituted with (=O), halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R, ⁶ -(CH₂)₀₋₃-(CO)-NH-R⁶, -(CH₂)₀₋₃-NH-(CO)-R⁶, 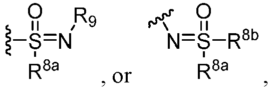
R⁶, R⁷, R^{8a}, R^{8b}, R⁹ and R¹⁰ are as defined above.

In some embodiments, R^{a} and R^{b} are H;

In some embodiments, R² is H;

In some embodiments, R²⁰ is H;

In some embodiments, R⁴ and R⁵ are each independently selected from H and C₁₋₆ alkyl;

In some embodiments, R⁴ and R⁵ are each independently selected from C₁₋₆ alkyl;

In some embodiments, R⁴ and R⁵ is H;

In some embodiments, R³ is selected from 5-12 membered heteroaryl, wherein the heteroaryl can be optionally substituted with halogen, -CN, -OH, -NH₂, NOz, -(CH₂)₀₋₆-CF₃, -O-C₁₋₆ alkyl, -NHC₁₋₆ alkyl, C₁₋₆ alkyl, 3-8 membered cycloalkyl, 3-8 membered heterocycloalkyl, 6-10 membered aryl, 5-12 membered heteroaryl, -(CH₂)₀₋₃-(CO)-C₁₋₆ alkyl, -(CH₂)₀₋₃-(CO)-NH-C₁₋₆ alkyl, or -(CH₂)₀₋₃-NH-(CO)-C₁₋₆ alkyl; preferably, the heteroaryl can be optionally substituted with halogen, -CN, -OH, -NH₂, -CF₃, -O-C₁₋₆ alkyl, -NHC₁₋₆ alkyl, C₁₋₆ alkyl, 3-8 membered cycloalkyl, 3-8 membered heterocycloalkyl, 6-10 membered aryl, or 5-12 membered heteroaryl; more preferably, the heteroaryl can be optionally substituted with halogen, -CN, -OH, -NH₂, -CF₃, -O-C₁₋₆ alkyl, -NHC₁₋₆ alkyl, or C₁₋₆ alkyl;

In some embodiments, R³ is 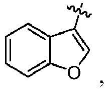 wherein the 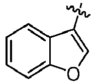 can be optionally substituted with halogen, -CN, -OH, -NH₂, NOz, -(CH₂)₀₋₆-CF₃, -O-C₁-₆ alkyl, -NHC₁₋₆ alkyl, C₁₋₆ alkyl, 3-8 membered cycloalkyl, 3-8 membered heterocycloalkyl, 6-10 membered aryl, 5-12 membered heteroaryl, -(CH₂)₀₋₃-(CO)-C₁₋₆ alkyl, -(CH₂)₀₋₃-(CO)-NH-C₁₋₆ alkyl, or -(CH₂)₀₋₃-NH-(CO)-C₁₋₆ alkyl; preferably, the 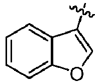 can be optionally substituted with halogen, -CN, -OH, -NH₂, -CF₃, -O-C₁₋₆ alkyl, -NHC₁₋₆ alkyl, C₁₋₆ alkyl, 3-8 membered cycloalkyl, 3-8 membered heterocycloalkyl, 6-10 membered aryl, or 5-12 membered heteroaryl; more preferably, the 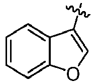 can be optionally substituted with halogen, -CN, -OH, -NH₂, -CF₃, -O-C₁₋₆ alkyl, -NHC₁₋₆ alkyl, or C₁₋₆ alkyl;

In some embodiments, R¹ is selected from C₁₋₆ alkyl, 3-12 membered cycloalkyl, 3-12 membered heterocycloalkyl, 6-10 membered aryl, and 5-12 membered heteroaryl, preferably selected from 3-8 membered cycloalkyl, 3-8 membered heterocycloalkyl, 6-10 membered aryl, and 5-12 membered heteroaryl, wherein the alkyl, cycloalkyl and heterocycloalkyl can be optionally substituted with (=O); the cycloalkyl, heterocycloalkyl, aryl and heteroaryl can be optionally substituted with halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 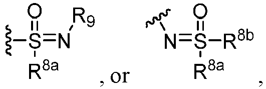 or,
R¹ and R²⁰ join together to form a 3-12 membered heterocycle, wherein the heterocycle can be optionally substituted with (=O), halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 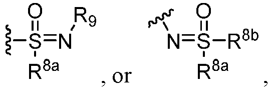
R⁶ are each independently selected from C₁₋₆ alkyl, 3-8 membered cycloalkyl, 3-8 membered heterocycloalkyl, 6-10 membered aryl, and 5-12 membered heteroaryl, preferably selected from C₁₋₆ alkyl and 3-8 membered cycloalkyl, more preferably selected from C₁₋₆ alkyl,
R¹⁰ are each independently selected from H and C₁₋₆ alkyl, preferably H,
R⁷ are each independently selected from 3-8 membered cycloalkyl, 3-8 membered heterocycloalkyl, 6-10 membered aryl, and 5-12 membered heteroaryl, preferably selected from 3-8 membered cycloalkyl and 3-8 membered heterocycloalkyl, wherein the cycloalkyl, heterocycloalkyl, aryl and heteroaryl can be optionally substituted with halogen, -CN, -OH, -NH₂, NOz, -O-C₁₋₆ alkyl, -N(C₁₋₆ alkyl) (C₁₋₆ alkyl), or C₁₋₆ alkyl,
R^{8a} and R^{8b} are each independently selected from C₁₋₆ alkyl, 3-8 membered cycloalkyl, 3-8 membered heterocycloalkyl, 6-10 membered aryl, and 5-12 membered heteroaryl, preferably selected from C₁₋₆ alkyl and 3-8 membered cycloalkyl, more preferably selected from C₁₋₆ alkyl, or
R^{8a} and R^{8b} can join together to form a 3-8 membered heterocycle, and
R⁹ is selected from H, C₁₋₆ alkyl, 3-8 membered cycloalkyl, 3-8 membered heterocycloalkyl, 6-10 membered aryl, and 5-12 membered heteroaryl, preferably selected from H, C₁₋₆ alkyl and 3-8 membered cycloalkyl, more preferably selected from H and C₁₋₆ alkyl;

In some embodiments, m is 0 or 1, preferably 0;

In some embodiments, n is 1;

In some embodiments, the present invention provides the following compound or a pharmaceutically acceptable salt, solvate, polymorph, or tautomer thereof:

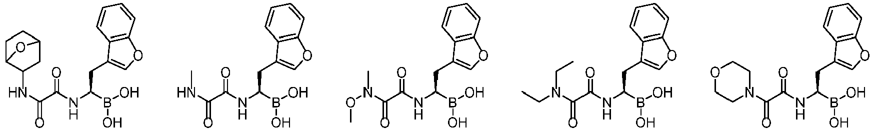

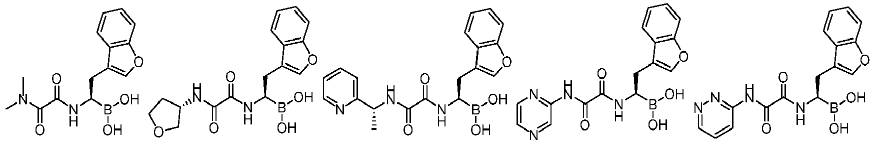

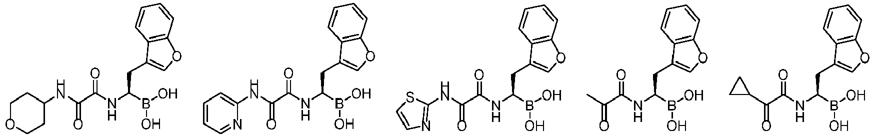

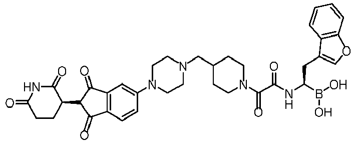

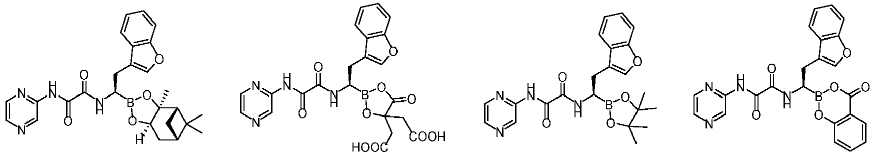

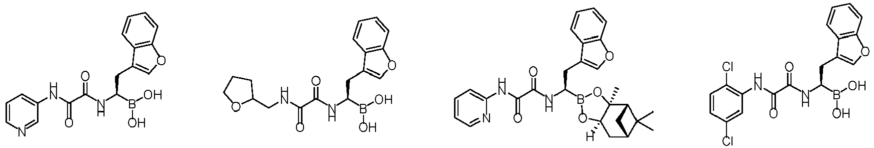

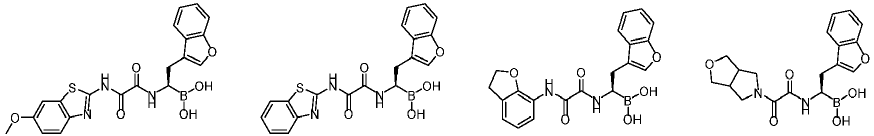

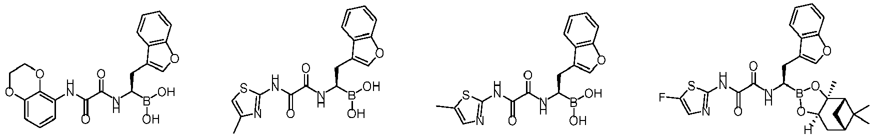

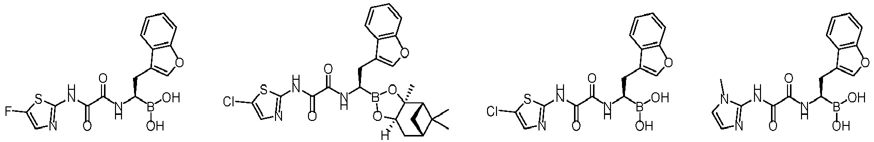

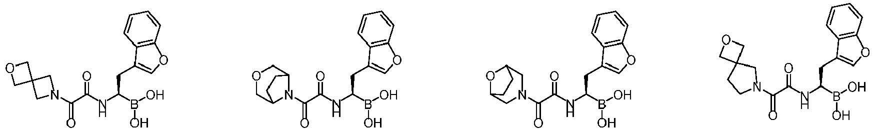

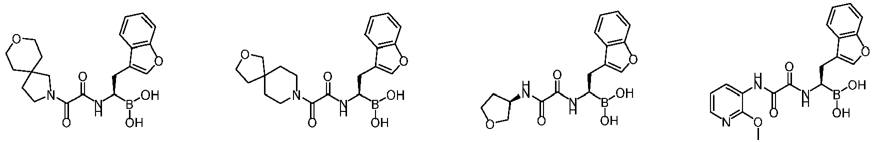

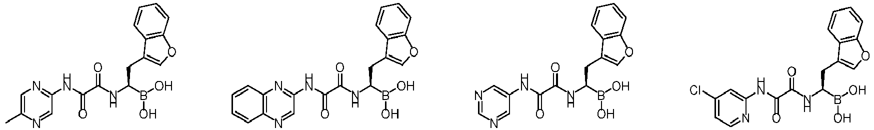

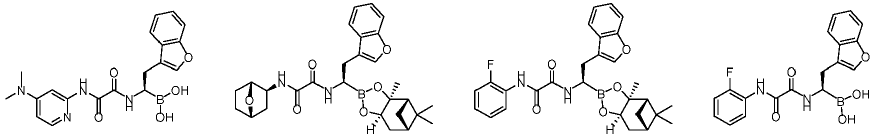

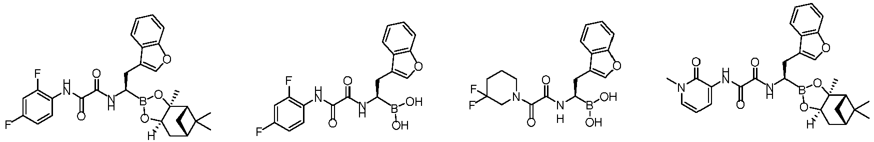

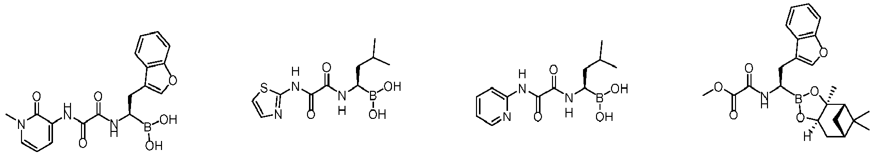

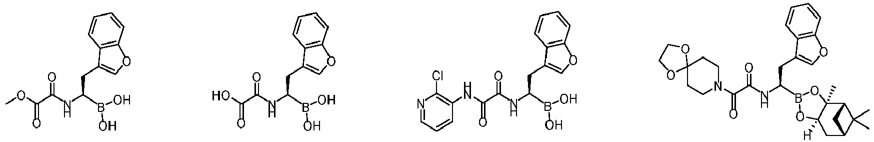

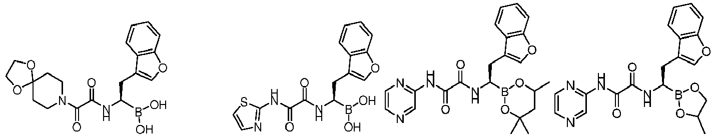

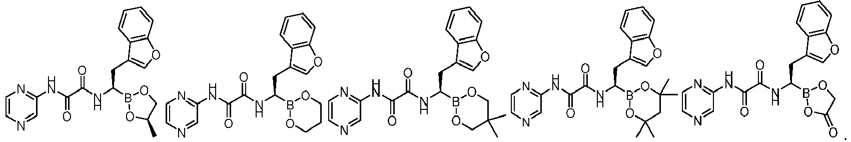

The compound of the invention, or the prodrug, ester, ether, solvate, polymorph, isomer or pharmaceutically acceptable salt of any of the foregoing, or a mixture of any two or more of the foregoing, in any ratio, can be used to treat lmp7-related diseases; in some embodiments, the lmp7 activity-related disease is multiple myeloma, acute myeloid leukemia, myeloid leukemia, mantle cell lymphoma, chronic lymphocytic leukemia, acute lymphocytic leukemia, diffuse large B-cell lymphoma, plasmacytoma, follicular lymphoma, immunocytoma, breast cancer, liver cancer, colorectal cancer, ovarian cancer, esophageal cancer, lung cancer, head and neck cancer, pancreatic cancer, renal cancer, gastric cancer, thyroid cancer, prostate cancer, bladder cancer, rheumatoid arthritis, systemic lupus erythematosus, inflammatory bowel disease, multiple sclerosis, scleroderma, ankylosing spondylitis, atherosclerosis, Behcet's disease, Crohn's disease, inflammatory bowel disease, ulcerative colitis, autoimmune hepatitis, Sjogren's syndrome, lupus nephritis, asthma, amyotrophic lateral sclerosis (ALS), psoriasis, immunoglobulin A nephropathy, allergic purpura, and Alzheimer's disease (AD);

Another aspect of the invention also relates to a pharmaceutical composition comprising the compound of the invention, or the prodrug, ester, ether, solvate, polymorph, isomer, or pharmaceutically acceptable salt of any of the foregoing, or a mixture of any two or more of the foregoing, in any ratio, and optionally a pharmaceutically acceptable carrier;

In another aspect, the present invention provides a method of treating lmp7 activity-related disease comprising administering to a subject an effective amount of the compound of the present invention, or the prodrug, ester, ether, solvate, polymorph, isomer or pharmaceutically acceptable salt of any of the foregoing, or a mixture of any two or more of the foregoing in any ratio, or a composition of the foregoing; in some embodiments, the lmp7 activity-related disease is multiple myeloma, acute myeloid leukemia, myeloid leukemia, mantle cell lymphoma, chronic lymphocytic leukemia, acute lymphocytic leukemia, diffuse large B-cell lymphoma, plasmacytoma, follicular lymphoma, immunocytoma, breast cancer, liver cancer, colorectal cancer, ovarian cancer, esophageal cancer, lung cancer, head and neck cancer, pancreatic cancer, renal cancer, gastric cancer, thyroid cancer, prostate cancer, bladder cancer, rheumatoid arthritis, systemic lupus erythematosus, inflammatory bowel disease, multiple sclerosis, scleroderma, ankylosing spondylitis, atherosclerosis, Behcet's disease, Crohn's disease, inflammatory bowel disease, ulcerative colitis, autoimmune hepatitis, Sjogren's syndrome, lupus nephritis, asthma, amyotrophic lateral sclerosis (ALS), psoriasis, immunoglobulin A nephropathy, allergic purpura, and Alzheimer's disease (AD);

In some embodiments of the invention, the subject to which the invention relates is a mammal, including a human;

In another aspect, the present invention provides the use of the compound of the present invention, or the prodrug, ester, ether, solvate, polymorph, isomer or pharmaceutically acceptable salt of any of the foregoing, or a mixture of any two or more of the foregoing in any ratio, in the manufacture of a medicament for the treatment of lmp7 activity-related diseases; in some embodiments, the lmp7 activity-related disease is multiple myeloma, acute myeloid leukemia, myeloid leukemia, mantle cell lymphoma, chronic lymphocytic leukemia, acute lymphocytic leukemia, diffuse large B-cell lymphoma, plasmacytoma, follicular lymphoma, immunocytoma, breast cancer, liver cancer, colorectal cancer, ovarian cancer, esophageal cancer, lung cancer, head and neck cancer, pancreatic cancer, renal cancer, gastric cancer, thyroid cancer, prostate cancer, bladder cancer, rheumatoid arthritis, systemic lupus erythematosus, inflammatory bowel disease, multiple sclerosis, scleroderma, ankylosing spondylitis, atherosclerosis, Behcet's disease, Crohn's disease, inflammatory bowel disease, ulcerative colitis, autoimmune hepatitis, Sjogren's syndrome, lupus nephritis, asthma, amyotrophic lateral sclerosis (ALS), psoriasis, immunoglobulin A nephropathy, allergic purpura, and Alzheimer's disease (AD).

### Brief Description of the Drawings

FIG. 1 shows the tumor-bearing mice growth curve and tumor-bearing mice body weight curve in *in-vivo* assay I;
FIG. 2 shows the tumor-bearing mice growth curve and tumor-bearing mice body weight curve in *in-vivo* assay II.

### Detailed Description of the Invention

Exemplary embodiments utilizing the principles of the present invention are set forth in the detailed description that follows. By referring to the following description, the features and advantages of the present invention can be better understood.

It should be understood that the scope for various aspects of the present invention is determined by the claims, and the methods and structures within the scope of these claims, as well as their equivalent methods and structures, are all within the scope of the present claims.

Unless otherwise defined, all technical terms herein have the same meanings as those commonly understood by those skilled in the field to which the subject matter of the claims belongs. Unless otherwise specified, all patents, patent applications, and public materials cited herein are incorporated by reference in their entirety.

It should be understood that the above description and the following description are exemplary and explanatory, and not limiting any of the subject matter of the present invention. The use of the singular also includes the plural unless specifically stated otherwise. Unless otherwise specified, the use of "or" represents "and/or". Furthermore, the use of the term "including" as well as other forms, such as "include", "includes," and "included," is not limiting.

### Certain Chemical Terminology

The terms "option", "optional", or "optionally" refer to events or situations that may or may not occur subsequently described, including the occurrence and non-occurrence of the events or situations. For example, "optionally substituted alkyl" means "unsubstituted alkyl" or "substituted alkyl". Also, optionally substituted groups may be unsubstituted (e.g., -CH₂CH₃), fully substituted (e.g., -CF₂CF₃), monosubstituted (e.g., -CH₂CH₂F), or any level between mono-and fully substituted (e.g., -CH₂CHF₂, -CF₂CH₃, -CFHCHF₂, and the like). It will be understood by those skilled in the art that for any group containing one or more substituents, no substitution or substitution pattern is introduced that is sterically impossible and/or synthetically non-feasible.

Unless otherwise indicated, conventional methods within the skill of the art are employed, such as, for example, mass spectrometry, nuclear magnetic resonance, high-performance liquid chromatography, infrared and ultraviolet/visible spectroscopy, and pharmacological methods. Unless a specific definition is provided, the relevant terms, experimental steps, and techniques in analytical chemistry, organic synthesis chemistry, and pharmaceutical and pharmaceutical chemistry are known in this field. Standard techniques can be used in chemical syntheses, chemical analyses, pharmaceutical preparation, formulation, delivery, and treatment of patients. For example, the reaction and purification can be carried out using the manufacturer's instructions for use of the kit, or in a manner known in the art or in the present invention. The techniques and methods described above can generally be carried out according to conventional methods well known in the art, in light of the description in the numerous general and more specific documents cited and discussed in this specification. In the description, groups, and substituents thereof are chosen by one skilled in the field to provide stable structure moieties and compounds.

When a substituent is described by a conventional chemical formula written from left to right, the substituent also includes chemically equivalent substituents that result when the structural formula is written from right to left. For example, -CHzO- is equivalent to -OCH₂-.

As used herein, the terms "group" and "chemical group" refer to a particular moiety or functional group of a molecule. Chemical groups are often recognized as chemical entities embedded or connected to a molecule.

Some of the chemical groups named herein may represent the total number of carbon atoms with a shorthand notation. For example, C₁-C₆ alkyl describes an alkyl group, as defined below, having a total of 1 to 6 carbon atoms. The total number of carbon atoms indicated by the shorthand notation does not include carbon atoms on possible substituents.

The terms "halogen", "halogenated", or "halide" refer to bromine, chlorine, fluorine, or iodine.

The terms "aromatic", "aromatic ring", "aromatic", and "aromatic cyclic", as used herein, refer to a planar ring or ring portion of a ring having a delocalized electron conjugate system containing 4n+2 electrons, where n is an integer. The aromatic ring may be formed from 5, 6, 7, 8, 9, or more than 9 atoms. Aromatic compounds may be optionally substituted and may be monocyclic or fused-ring polycyclic. The term aromatic compounds includes all carbon rings (such as benzene rings) and rings containing one or more heteroatoms (such as pyridine).

The term "heteroatom" or "hetero" as used herein alone or as part of another component refers to an atom other than carbon and hydrogen. Heteroatoms are independently selected from oxygen, nitrogen, sulfur, phosphorus, silicon, selenium, and tin, but are not limited to these atoms. In embodiments where two or more heteroatoms are present, the two or more heteroatoms may be the same as each other, or some or all of the two or more heteroatoms may be different from each other.

The term "bridged ring" as used herein, alone or in combination, refers to a cyclic structure in which any two rings in a compound share two carbon atoms that are not directly connected.

The term "fused" or "fused ring" as used herein, alone or in combination, refers to a cyclic structure in which two or more rings share one or more bonds.

The term "spiro" or "spirocycle" as used herein, alone or in combination, refers to a cyclic structure in which two or more rings share one or more atoms.

Alone or as part of another component (e.g., monoalkylamino), the term "alkyl" as used herein refers to an optionally substituted liner or branched chain monovalent saturated hydrocarbon radical having 1-12 carbon atoms, preferably 1-8 carbon atoms, more preferably 1-6 carbon atoms, connected to the rest of the molecule by a single bond, e.g. methyl, ethyl, n-propyl, isopropyl, n-butyl, isobutyl, sec-butyl, tert-butyl, n-pentyl, n-hexyl, n-heptyl, 2-methylhexyl, 3-methylhexyl, n-octyl, n-nonyl, n-decyl and the like.

The term "cycloalkyl", as used herein alone or as part of another component, refers to a stable monovalent non-aromatic monocyclic or multicyclic hydrocarbon radical, consisting solely of carbon and hydrogen atoms, which may include fused, spiro, or bridged ring systems, containing 3-15 ring carbon atoms, preferably 3-10 ring carbon atoms, more preferably 3-8 ring carbon atoms, which may be saturated or unsaturated, and which may be connected to the rest of the molecule by a single bond. Non-limiting examples of "cycloalkyl" include but are not limited to cyclopropyl, cyclobutyl, cyclopentyl, cyclohexyl, and cyclohexyl.

The terms "heterocyclyl", "heterocycloalkyl", "heterocycle", as used herein alone or as part of another component, refer to a stable 3-18 membered monovalent non-aromatic ring that contains 2-12 carbon atoms and 1-6 heteroatoms selected from nitrogen, oxygen, and sulfur. Unless otherwise specified, the heterocyclyl group may be a monocyclic, bicyclic, tricyclic, or tetracyclic ring system, which may contain fused, spiro, or bridged ring systems; the nitrogen, carbon, or sulfur of the heterocyclyl group may be optionally oxidized, the nitrogen atom may be optionally quaternized, and the heterocyclyl group may be partially or fully saturated. A heterocyclyl group can be connected to the remainder of the molecule through a single bond via a carbon atom or heteroatom in the ring. Heterocyclyl groups containing fused rings may contain one or more aromatic or heteroaromatic rings provided that the atoms on the non-aromatic ring to which the remainder of the molecule is connected. For purposes of this application, heterocyclyl is preferably a stable 4-11 membered monovalent non-aromatic monocyclic or bicyclic ring containing 1-3 heteroatoms selected from nitrogen, oxygen, and sulfur, more preferably a stable 4-8 membered monovalent non-aromatic monocyclic ring containing 1-3 heteroatoms selected from nitrogen, oxygen and sulfur. Non-limiting examples of heterocyclyl groups include azepanyl, azetidinyl, decahydroisoquinolinyl, dihydrofuranyl, indolinyl, dioxolanyl, 1,1-dioxo-thiomorpholinyl, imidazolidinyl, imidazolinyl, isothiazolidinyl, isoxazolidinyl, morpholinyl, octahydroindolyl, octahydroisoindolyl, oxazinyl, piperazinyl, piperidinyl, 4-piperidonyl, pyranyl, pyrazolidinyl, pyrrolidinyl, quinolizinyl, quinuclidinyl, tetrahydrofuranyl, tetrahydropyranyl, and the like.

The term "aryl" refers to an all-carbon monocyclic or fused ring having a fully conjugated π-electron system having 6-14 carbon atoms, preferably 6-12 carbon atoms, most preferably 6 carbon atoms. Aryl groups can be unsubstituted or substituted with one or more substituents, examples of which include, but are not limited to, alkyl, alkyloxy, aryl, aralkyl, amino, halogen, hydroxy, sulfonyl, sulfinyl, phosphoryl, and heteroalicyclyl. Non-limiting examples of non-substituted aryl groups include but are not limited to phenyl, naphthyl, and anthracene groups.

The term "heteroaryl" refers to a single or fused ring with 5-12 ring atoms, consisting of 5, 6, 7, 8, 9, 10, 11, or 12 ring atoms, containing 1, 2, 3, or 4 ring atoms selected from N, O, S, the remaining ring atoms being C, and having a completely conjugated π - electron system. Heteroaryl groups can be unsubstituted or substituted, examples of which include, but are not limited to, alkyl, alkyloxy, aryl, aralkyl, amino, halogen, hydroxy, cyano, nitro, carbonyl, and heteroalicyclyl. Non-limiting examples of unsubstituted heteroaryl groups include but are not limited to, pyrrolyl, furanyl, thienyl, imidazolyl, oxazolyl, pyrazolyl, pyridinyl, pyrimidinyl, pyrazinyl, quinolinyl, isoquinolinyl, tetrazolyl, and triazinyl.

As used herein, the term "polymorph" or "polymorphism" means that the compounds of the present invention have multiple crystal lattice morphologies. Some of the compounds of the invention may have more than one crystalline form, and the invention encompasses all polymorphs or mixtures thereof.

Intermediate compounds of the compounds of the invention and polymorphs thereof are also within the scope of the invention.

Unless otherwise specified, olefinic double bonds contained in the compounds of the present invention include *E* and *Z* isomers.

It will be appreciated that the compounds of the present invention may contain asymmetric centers. These asymmetric centers may independently be in the *R* or *S* configuration. Some compounds of the present invention may also exhibit *cis-trans* isomerism, which is evident to those skilled in the art. It will be understood that the compounds of the present invention include their individual geometric isomers and stereoisomers as well as mixtures thereof, including racemic mixtures. These isomers can be separated from their mixtures by the implementation or modification of known methods, for example chromatographic techniques and recrystallization techniques, or they can be prepared separately from the appropriate isomers of their intermediates.

As used herein, the term "pharmaceutically acceptable salts" includes both acid and base addition salts.

"Pharmaceutically acceptable acid addition salt" refers to those salts which retain the biological effectiveness and properties of the free bases of the compounds and which are not biologically or otherwise undesirable, formed with inorganic acids such as, but not limited to, hydrochloric acid, hydrobromic acid, sulfuric acid, nitric acid, phosphoric acid and the like, or organic acids such as, but not limited to, acetic acid, 2,2-dichloroacetic acid, adipic acid, alginic acid, ascorbic acid, aspartic acid, benzene sulfonic acid, benzoic acid, decanoic acid, hexanoic acid, carbonic acid, cinnamic acid, citric acid and the like. "Pharmaceutically acceptable base salt" refers to those salts which retain the biological effectiveness and properties of the free acids of the compounds and which are not biologically or otherwise undesirable. These salts are prepared by reacting the free acid with an inorganic or organic base. Salts formed by reaction with inorganic bases include but are not limited to, sodium, potassium, lithium, ammonium, calcium, magnesium, iron, zinc, copper, manganese, aluminum salts, and the like. Preferred inorganic salts are ammonium, sodium, potassium, calcium, and manganese salts.

Salts forming organic bases include but are not limited to, primary, secondary, tertiary, cyclic amines and the like, such as ammonia, isopropylamine, trimethylamine, diethylamine, triethylamine, tripropylamine, ethanolamine, diethanolamine, ethanolamine, dicyclohexylamine, ethylenediamine, purine, piperazine, piperidine, choline, caffeine and the like. Particularly preferred organic bases are isopropylamine, diethylamine, ethanolamine, trimethylamine, dicyclohexylamine, choline, and caffeine.

Crystallization often produces solvates of compounds of the present invention. As used herein, the term "solvate" refers to an assembly of one or more molecules of a compound of the invention with one or more molecules of a solvent.

The solvent may be water, in which case the solvate is a hydrate. Organic solvents are also possible. Thus, the compounds of the present invention may exist as a hydrate, including a monohydrate, dihydrate, hemihydrate, trihydrate, tetrahydrate, and the like, as well as the corresponding solvated forms. The compounds of the present invention may be true solvates, but in other cases the compounds of the present invention may only occasionally retain water or a mixture of water with some other solvent. The compounds of the present invention can be reacted in a solvent or precipitated or crystallized in a solvent. Solvates of the compounds of the present invention are also included within the scope of the present invention.

As used herein, the term "pharmaceutical composition" refers to a formulation incorporating a compound of the present invention and a medium generally accepted in the art for the delivery of biologically active compounds to mammals, such as humans. This medium contains all pharmaceutically acceptable carriers.

The term "acceptable" used herein regarding formulations, compositions, or ingredients refers to the absence of sustained harmful effects on the overall health of the subject to be treated.

The term "pharmaceutically acceptable" used in herein refers to a substance (such as a carrier or diluent) that does not affect the biological activity or properties of the compound of the present invention and is relatively non-toxic, meaning that the substance can be applied to an individual without causing adverse biological reactions or interacting with any component contained in the composition in an adverse manner.

"Pharmaceutically acceptable carrier" includes, but is not limited to, adjuvants, carriers, excipients, adjuvants, deodorants, diluents, preservatives, dyes/colorants, flavor enhancers, surfactants and wetting agents, dispersants, suspending agents, stabilizers, isotonic agents, solvents, or emulsifiers that have been approved by the relevant governmental agency to be used with humans and domestic animals.

As used herein, the term "subject", "patient", or "individual" refers to an individual suffering from a disease, disorder, or condition, and the like, including mammals and non-mammals. Examples of mammals include but are not limited to any member of the mammalian class: humans, non-human primates (such as chimpanzees and other apes and monkeys); livestock, such as cows, horses, sheep, goats, pigs; domestic animals, such as rabbits, dogs, and cats; experimental animals, including rodents such as rats, mice, and guinea pigs. Examples of non-human mammals include but are not limited to birds and fish. In one embodiment of the methods and compositions provided herein, the mammal is a human.

The term "treatment" used herein refers to the treatment of diseases or conditions related to mammals, especially humans, including
(i) preventing a mammal, particularly a mammal that has previously been exposed to a disease or condition but has not yet been diagnosed as having it, from developing the corresponding disease or condition;
(ii) inhibiting the disease or disorder, i.e. controlling its development;
(iii) relieving the disease or condition, i.e. causing regression of the disease or condition; and
(iv) alleviating the symptoms caused by the disease or disorder.

As used herein, the terms "disease" and "condition" may be used interchangeably or in a different sense, because certain specific diseases or conditions do not yet have a known causative agent (so the cause of the disease is unknown), and therefore cannot be considered a disease but only an unwanted condition or syndrome whose more or less specific symptoms have been confirmed by clinical investigators.

The terms "effective amount", "therapeutically effective amount", or "pharmaceutically effective amount", as used herein, refer to an amount of at least one agent or compound that, upon administration, is sufficient to relieve to some extent one or more of the symptoms of the disease or disorder being treated. The result may be reduction and/or alleviation of the signs, symptoms, or causes of a disease, or any other desired alteration of a biological system. For example, the "effective amount" used for treatment is the amount of composition containing the compounds disclosed herein required to provide significant symptom relief in clinical practice. An effective amount in any individual case may be determined using techniques, such as a dose escalation assay.

The terms "administration", "administrate", "administrating", etc. used herein refer to methods that can deliver a compound or combination to the desired site for biological action. These methods include, but are not limited to oral routes, intraduodenal routes, parenteral injection (including intravenous, subcutaneous, intraperitoneal, intramuscular, intravascular injection, or infusion), topical and rectal administration. In the preferred embodiment, the compounds and compositions discussed herein are administered orally.

### DESCRIPTION

### Synthetic method

The compound of the present disclosure can be prepared according to the routes described in Scheme 1, scheme 2, scheme 3, or Scheme 4.

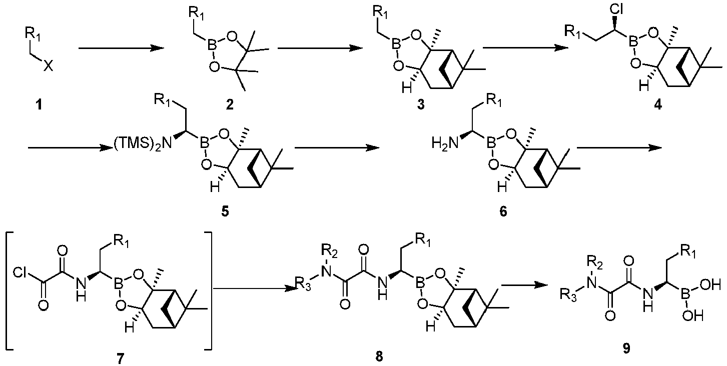

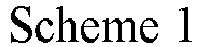

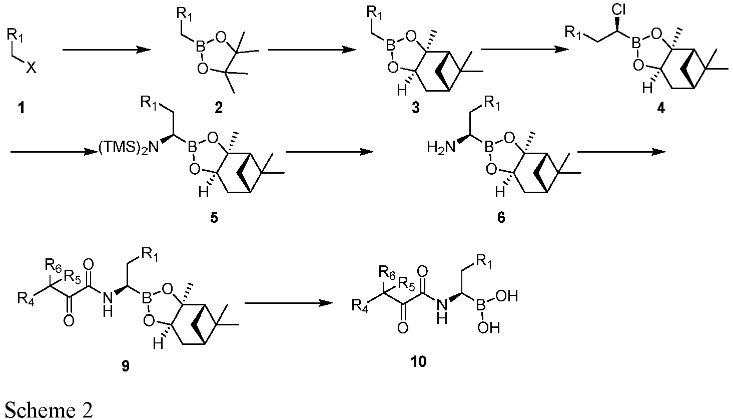

In Schemes 1 and 2, R₁, R₂, R₃, R₄, R₅ and R₆ substituents include, but are not limited to, hydrogen atoms, aliphatic, and aromatic substituents; X includes but is not limited to, fluoro, chloro, bromo, iodo, triflate, and the like. Each of the products resulting from the reactions in Schemes 1 and 2 may be obtained by conventional separation techniques including, but not limited to, filtration, distillation, crystallization, chromatography, and the like. Starting materials can be synthesized by themselves or purchased from commercial establishments such as, but not limited to, Aldrich or Sigma. Such materials can be characterized using conventional means, such as physical constants and spectral data. The compounds described herein can be obtained as single isomers or as mixtures of isomers using the synthetic method.

In Scheme 1, starting material 1 undergoes a Suzuki reaction with bis (pinacolato) diboron in the presence of a copper catalyst, a ligand, and a suitable base to generate intermediate 2; intermediate 2 undergoes a transesterification with pinanediol to generate an optically active intermediate 3; intermediate 3 undergoes a Matteson homologation reaction with a pre-prepared LiCHCl₂ reagent under the catalysis of a zinc reagent to generate intermediate 4; intermediate 4 undergoes a substitution with a salt containing hexamethyldisilicylamine to generate intermediate 5; intermediate 5 removes the TMS group in the presence of an acid to generate intermediate 6; intermediate 6 undergoes a condensation with oxalyl chloride in the presence of a base to generate oxamide intermediate 7; intermediate 7 reacts with a substituted amine to generate oxamide intermediate 8, and intermediate 8 undergoes transesterification with excess substituted boronic acid to generate target compound 9.

In Scheme 2, starting material 1 undergoes a Suzuki reaction with bis (pinacolato) diboron in the presence of a copper catalyst, a ligand and a suitable base to generate intermediate 2; intermediate 2 undergoes a transesterification with pinanediol to generate optically active intermediate 3; intermediate 3 undergoes a Matteson homologation reaction with a pre-prepared LiCHCl₂ reagent under the catalysis of a zinc reagent to generate intermediate 4; intermediate 4 undergoes a substitution with a salt containing hexamethyldisilazane to generate intermediate 5; intermediate 5 removes the TMS group in the presence of an acid to generate intermediate 6; intermediate 6 reacts with a substituted 2-oxo acid chloride in the presence of a base to generate 2-oxoamide intermediate 10, and intermediate 10 undergoes a transesterification with excess substituted boronic acid to generate target compound 11.

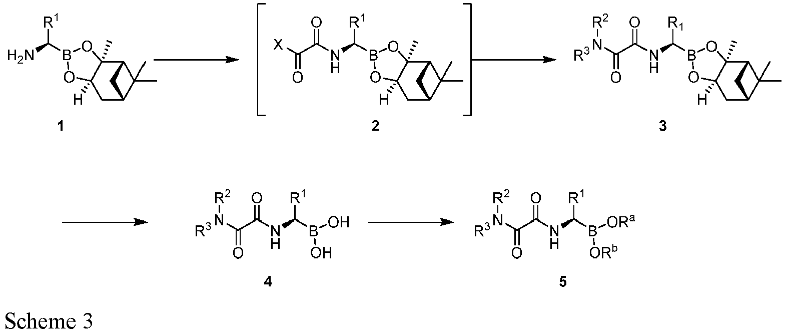

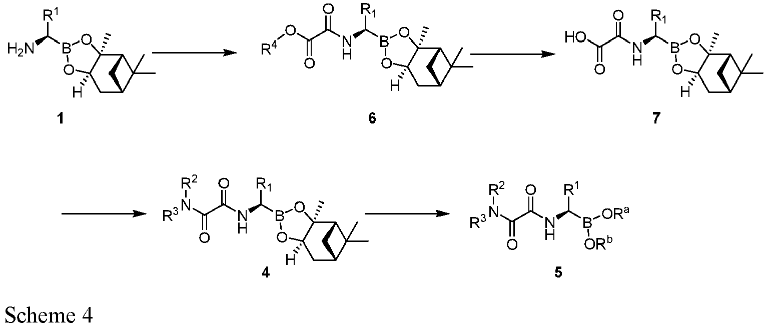

In Schemes 3 and 4, R¹, R², R³, R⁴, R^{a}, and R^{b} substituents include, but are not limited to, hydrogen atoms, aliphatic and aromatic substituents; X includes but is not limited to, fluoro, chloro, bromo, iodo, triflate, and the like. R^{a} and R^{b} may be joined to form a ring, a bridged ring, a fused ring, or an aromatic fused ring. Each of the products resulting from the reactions in Schemes 1 and 2 may be obtained by conventional separation techniques including, but not limited to, filtration, distillation, crystallization, chromatography, and the like. Starting materials can be synthesized by themselves or purchased from commercial establishments such as, but not limited to, Aldrich or Sigma. Such materials can be characterized using conventional means, such as physical constants and spectral data. The compounds described herein can be obtained as single isomers or as mixtures of isomers using the synthetic method.

In Scheme 3, starting material 1 undergoes a condensation with oxalyl chloride in the presence of a base to generate oxamide intermediate 2; intermediate 2 reacts with substituted amine to generate oxamide intermediate 3; intermediate 3 undergoes a transesterification with excess substituted boronic acid to generate compound 4, and compound 4 reacts with carboxylic acid or alcohol to generate prodrug compound 5.

In Scheme 4, starting material 1 reacts with oxalyl chloride monomethyl ester under a basic condition to generate compound 6; compound 6 is hydrolyzed under basic conditions to generate intermediate 7; intermediate 7 reacts with a substituted amine to generate compound 4; compound 4 undergoes transesterification with excess substituted boronic acid to generate target compound 5.

### Example 1: ((R)-1-(2-(((1S,2R,4R)-7-oxabicyclo[22.1]heptan-2-yl)amino)-2-oxoacet amido)-2-(benzofuran-3-yl)ethyl)boronic acid

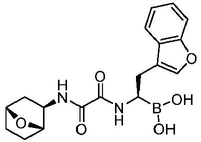

### Step A: (1S,2R,4R)-7-oxabicyclo[2.2.1]heptan-2-amine

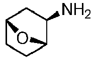

(1*S*,2*R*,4*R*)-7-oxabicyclo[22.1]heptan-2-carboxylic acid (obtained according to the method in patent WO 2019/038250 A1) (1.0 g) and triethylamine (0.75 g) were mixed in toluene (10 mL), the mixture was stirred at 25 °C for 30 minutes, diphenyl phosphoryl azide (2.05 g) was added, the mixture was stirred at room temperature for 2 hours, water (0.3 g) was added, and the mixture was heated to 70 °C and stirred overnight. The mixture was cooled to room temperature, water (10 mL) was added, the organic phase was separated, 4 mol/L hydrochloric acid (15 mL) was added, the mixture was sufficiently stirred, the water phase was separated and washed with ethyl acetate (15 mL x 2), 4 mol/L aqueous sodium hydroxide solution was used to adjust the pH to 10, the mixture was extracted with dicholoromethane (15 mL x 3), the extracts were combined, dried over anhydrous sodium sulphate and filtered, and the filtrate was concentrated under reduced pressure to obtain the product (0.23 g).

¹H NMR (400 MHz, CDCl₃) δ 4.71 (d, *J* = 8.4 Hz, 1H), 4.32 (d, *J* = 5.2 Hz, 1H), 2.05-2.35 (brs, 2H), 1.96 (dd, *J* = 13.2 Hz, 8.0 Hz, 1H), 1.57-1.70 (m, 3H), 1.31-1.45 (m, 3H).

### Step B: ((R)-1-(2-(((1S,2R,4R)-7-oxabicyclo[2.2.1]heptan-2-yl)amino)-2-oxoacetamido)-2-(benzofuran-3-yl)ethyl)boronic acid (1S,2S,3R,5S)-(+)-pinane-2,3-diol ester

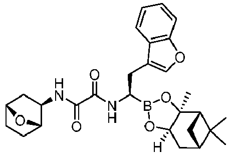

At 0 °C, to a solution of oxalyl chloride (250 mg) in dicholoromethane (5 mL) were sequentially added dropwise a solution of 2-(benzofuran-3-yl)-1-(*R*)-aminoethylboronic acid *(1S,2S,3R,5S)*-(+)-pinane-2,3-diol ester hydrochloride (obtained according to the method in patent WO 2019/038250 A1) (75 mg) in dicholoromethane (5 mL) and diisopropylethylamine (80 mg), the mixture was let warm up to room temperature, stirred for 2 hours, and concentrated under reduced pressure, and the residue was dissolved in dicholoromethane (10 mL). To this solution was added dropwise a solution of (1*S*,2*R*,4*R*)-7-oxabicyclo[2.2.1]heptan-2- amine (23 mg) in dicholoromethane (5 mL), the mixture was stirred at room temperature for 4 hours, 1 mol/L hydrochloride (5 mL) was added to quench the reaction, the organic phase was separated, washed with brine, dried over anhydrous sodium sulphate, and filtered, the filtrate was concentrated under reduced pressure, and the residue was purified through silica gel Preparative Thin-Layer Chromatography (1:1 ethyl acetate/petroleum ether) to obtain the product (53 mg).

¹H NMR (400 MHz, CDCl₃) δ 7.62 (d, *J* = 6.4 Hz, 1H), 7.55 (d, *J* = 7.2 Hz, 1H), 7.52 (d, *J* = 8.8 Hz, 1H), 7.45 (s, 1H), 7.42 (d, *J* = 8.0 Hz, 1H), 7.18-7.28 (m, 2H), 4.62 (t, *J* = 9.2 Hz, 1H), 4.36 (d, *J* = 5.2 Hz, 1H), 4.27 (dd, *J* = 8.8 Hz, 1.6 Hz, 1H), 4.01 (td, *J* = 8.4 Hz, 2.8 Hz, 1H), 3.67-3.72 (m, 1H), 3.11 (dd, *J* = 14.8 Hz, 5.6 Hz, 1H), 3.00 (dd, *J* = 15.2 Hz, 7.2 Hz, 1H), 2.19-2.33 (m, 2H), 2.06-2.12 (m, 1H), 1.95-2.02 (m, 1H), 1.80-1.91 (m, 2H), 1.60-1.74 (m, 3H), 1.42-1.55 (m, 2H), 1.38 (s, 3H), 1.22-1.24 (m, 3H), 0.99 (t, *J* = 4.0 Hz, 1H), 0.78 (s, 3H).

### Step C: ((R)-1-(2-(((1S,2R,4R)-7-oxabicyclo[2.2.1]heptan-2-yl)amino)-2-oxoacetamido)-2-(benzofuran-3-yl)ethyl)boronic acid

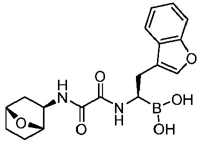

((*R*)-1-(2-(((1*S*,2*R*,4*R*)-7-oxabicyclo[2.2.1]heptan-2-yl)amino)-2-oxoacetamido)-2-(benz ofuran-3-yl)ethyl)boronic acid *(1S,2S,3R,5S)*-(+)-pinane-2,3-diol ester (53 mg) was dissolved in methanol (10 mL), to the solution were added isobutylboronic acid (52 mg), 1 mol/L hydrochloric acid (0.1 mL) and n-hexane (10 mL), and the mixture was stirred at room temperature overnight. The methanol phase was separated, washed with n-hexane, and concentrated under reduced pressure, and the residue was recrystallized from dicholoromethane/ethyl ether to obtain the product (23 mg).

¹H NMR (400 MHz, CD₃OD) δ 7.57 (d, *J* = 7.2 Hz, 1H), 7.52 (s, 1H), 7.40 (d, *J* = 8.0 Hz, 1H), 7.17-7.27 (m, 2H), 4.60 (t, *J* = 4.8 Hz, 1H), 4.34 (d, *J* = 5.2 Hz, 1H), 3.91 (dd, *J* = 8.0 Hz, 3.2 Hz, 1H), 3.53 (t, *J* = 7.2 Hz, 1H), 3.03 (dd, *J* = 14.8 Hz, 6.8 Hz, 1H), 2.93 (dd, *J* = 14.8 Hz, 7.2 Hz, 1H), 1.98 (dd, *J* = 12.8 Hz, 8.4 Hz, 1H), 1.58-1.66 (m, 3H), 1.42-1.53 (m, 2H), 1.34-1.37 (m, 1H).

### Example 2: (R)-(2-(benzofuran-3-yl)-1-(2-(methylamino)-2-oxoacetamido)ethyl)boronic acid

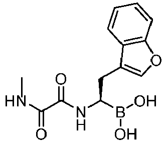

According to the method of step B and step C of example 1, 30% methylamine methanol solution, oxalyl chloride and 2-(benzofuran-3-yl)-1-(*R*)-aminoethylboronic acid *(1S,2S,3R,5S)-* (+)-pinane-2,3-diol ester hydrochloride were used as the starting material to obtain the product (15 mg).

¹H NMR (400 MHz, CDCl₃) δ 7.61 (d, *J* = 7.6 Hz, 1H), 7.55 (s, 1H), 7.40 (d, *J* = 8.0 Hz, 1H), 7.17-7.27 (m, 2H), 3.51 (t, *J* = 7.2 Hz, 1H), 3.12 (dd, *J* = 14.4 Hz, 7.2 Hz, 1H), 2.92 (dd, *J* = 14.48 Hz, 6.4 Hz, 1H), 2.90 (s, 3H).

### Example 3: (R)-(2-(benzofuran-3-yl)-1-(2-(methoxy(methyl)amino)-2-oxoacetamido)ethyl) boronic acid

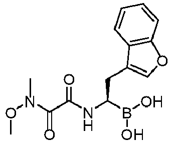

According to the method of step B and step C of example 1, *N*,*O*-dimethylhydroxylamine hydrochloride, diisopropylethylamine, oxalyl chloride and 2-(benzofuran-3-yl)-1-(*R*)-amino ethylboronic acid *(1S,2S,3R,5S)*-(+)-pinane-2,3-diol ester hydrochloride were used as the starting material to obtain the product (21 mg).

¹H NMR (400 MHz, CDCl₃) δ 7.58-7.62 (m, 1H), 7.53 (s, 1H), 7.41 (d, *J* = 8.0 Hz, 1H), 7.18-7.27 (m, 2H), 3.90 (s, 3H), 3.49-3.54(m, 1H), 3.33 (s, 3H), 3.03 (dd, *J* = 14.8 Hz, 6.8 Hz, 1H), 2.93 (dd, *J* = 14.8 Hz, 7.2 Hz, 1H).

### Example 4: (R)-(2-(benzofuran-3-yl)-1-(2-(diethylamino)-2-oxoacetamido)ethyl)boronic acid

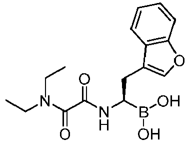

According to the method of step B and step C of example 1, diethylamine hydrochloride, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (18 mg).

¹H NMR (400 MHz, CD₃OD) δ 7.62 (d, *J* = 7.2 Hz, 1H), 7.58 (s, 1H), 7.42 (d, *J* = 8.0 Hz, 1H), 7.20-7.28 (m, 2H), 3.33-3.39 (m, 1H), 3.25-3.30 (m, 4H), 2.89-3.04 (m, 2H), 1.11-1.14 (m, 6H).

### Example 5: (R)-(2-(benzofuran-3-yl)-1-(2-morpholino-2-oxoacetamido)ethyl)boronic acid

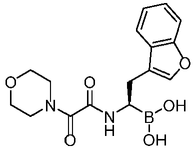

According to the method of step B and step C of example 1, morpholine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (25 mg).

¹H NMR (400 MHz, CD₃OD) δ 7.62 (d, *J* = 8.4 Hz, 1H), 7.57 (s, 1H), 7.42 (d, *J* = 8.0 Hz, 1H), 7.20-7.28 (m, 2H), 3.61-3.63 (m, 2H), 3.49-3.53 (m, 4H), 3.28-3.35 (m, 3H), 3.02 (dd, *J* = 14.8 Hz, 6.4 Hz, 1H), 2.92 (dd, *J* = 14.8 Hz, 8.8 Hz, 1H).

### Example 6: (R)-(2-(benzofuran-3-yl)-1-(2-(dimethylamino)-2-oxoacetamido)ethyl)boronic acid

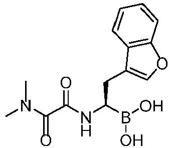

According to the method of step B and step C of example 1, diethylamine hydrochloride, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (33 mg).

¹H NMR (400 MHz, CD₃OD) δ 7.62 (d, *J* = 7.2 Hz, 1H), 7.57 (s, 1H), 7.41 (d, *J* = 8.4 Hz, 1H), 7.19-7.28 (m, 2H), 3.29-3.30 (m, 1H), 2.98-3.10 (m, 2H), 2.92 (s, 3H), 2.91 (s, 3H).

### Example 7: ((R)-2-(benzofuran-3-yl)-1-(2-oxo-2(((S)-tetrahydrofuran-3-yl)amino) acetamido)ethyl) boronic acid

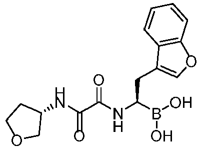

According to the method of step B and step C of example 1, (*S*)-3-aminotetrahydrofuran p-toluenesulphonic acid salt, diisopropylethylamine, oxalyl chloride and 2-(benzofuran-3-yl)-1- (*R*)-aminoethylboronic acid *(1S,2S,3R,5S)*-(+)-pinane-2,3-diol ester hydrochloride were used as the starting material to obtain the product (8 mg).

¹H NMR (400 MHz, CD₃OD) δ 7.58 (d, *J* = 7.2 Hz, 1H), 7.53 (s, 1H), 7.41 (d, *J* = 8.4 Hz, 1H), 7.18-7.27 (m, 2H), 4.35-4.41 (m, 1H), 3.73-3.95 (m, 3H), 3.51-3.65 (m, 2H), 3.00-3.09 (m, 1H), 2.94 (dd, *J* = 14.4 Hz, 7.6 Hz, 1H), 2.18-2.27 (m, 1H), 1.88-1.95 (m, 1H).

### Example 8: ((R)-2-(benzofuran-3-yl)-1-(2-oxo-2-(((R)-1-(pyridin-2-yl)ethyl)amino) acetamido)ethyl)boronic acid

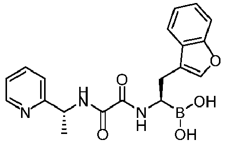

According to the method of step B and step C of example 1, (*R*)-1-(pyridin-2-yl)ethyl amine, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (10 mg).

¹H NMR (400 MHz, CD₃OD) δ 8.76 (d, *J* = 3.6 Hz, 1H), 8.62 (t, *J* =7.6 Hz, 1H), 7.99-8.12 (m, 2H), 7.56 (d, *J* = 8.0 Hz, 1H), 7.51 (s, 1H), 7.40 (d, *J* = 8.0 Hz, 1H), 7.13-7.26 (m, 2H), 5.22-5.28 (m, 1H), 3.54-3.59 (m, 1H), 3.05 (dd, *J* = 14.8 Hz, 6.4 Hz, 1H), 2.94 (dd, *J* = 14.4 Hz, 8.0 Hz, 1H), 1.68 (d, *J* = 6.8 Hz, 3H).

### Example 9: (R)-(2-(benzofuran-3-yl)-1-(2-oxo-2-(pyrazin-2-ylamino)acetamido)ethyl) boronic acid

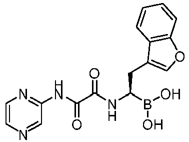

According to the method of step B and step C of example 1, aminopyrazine, diisopropyl ethylamine, oxalyl chloride and 2-(benzofuran-3-yl)-1-(*R*)-aminoethylboronic acid *(1S,2S,3R, 5S*)-(+)-pinane-2,3-diol ester hydrochloride were used as the starting material to obtain the product (13 mg).

¹H NMR (400 MHz, CD₃OD) δ 9.43 (s, 1H), 8.69 (s, 1H), 8.48 (s, 1H), 7.59 (d, *J* = 7.6 Hz, 1H), 7.54 (s, 1H), 7.38 (d, *J* = 8.0 Hz, 1H), 7.15-7.24 (m, 2H), 3.64-3.67 (m, 1H), 3.08 (dd, *J* = 14.4 Hz, 6.4 Hz, 1H), 2.99 (dd, *J* = 14.4 Hz, 8.0 Hz, 1H).

### Example 10: (R)-(2-(benzofuran-3-yl)-1-(2-oxo-2-(pyridazin-3-ylamino)acetamido)ethyl) boronic acid

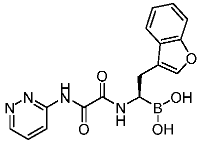

According to the method of step B and step C of example 1, 3-aminopyridazine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (15 mg).

¹H NMR (400 MHz, CD₃OD) δ 8.38-8.39 (m, 1H), 7.75-7.78 (m, 1H), 7.57 (d, *J* = 7.2 Hz, 1H), 7.51 (s, 1H), 7.40 (d, *J* = 8.0 Hz, 1H), 7.31-7.34 (m, 1H), 7.17-7.27 (m, 2H), 3.49-3.53 (m, 1H), 3.02 (dd, *J* = 14.4 Hz, 6.4 Hz, 1H), 2.94 (dd, *J* = 14.8 Hz, 7.2 Hz, 1H).

### Example 11: (R)-(2-(benzofuran-3-yl)-1-(2-oxo-2-((tetrahydro-2H-pyran-4-yl)amino) acetamido)ethyl)boronic acid

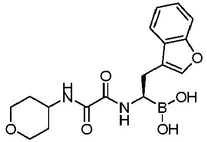

According to the method of step B and step C of example 1, 4-aminotetrahydropyran, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (7 mg).

¹H NMR (400 MHz, CD₃OD) δ 7.58 (d, *J* = 7.2 Hz, 1H), 7.52 (s, 1H), 7.41 (d, *J* = 8.0 Hz, 1H), 7.17-7.27 (m, 2H), 3.82-3.99 (m, 3H), 3.42-3.59 (m, 3H), 3.03 (dd, *J* = 14.4 Hz, 6.4 Hz, 1H), 2.93 (dd, *J* = 14.8 Hz, 7.2 Hz, 1H), 1.72-1.78 (m, 2H), 1.58-1.67 (m, 2H).

### Example 12: (R)-(2-((benzofuran-3-yl)-1-(2-oxo-2-(pyridin-2-ylamino)acetamido)ethyl) boronic acid

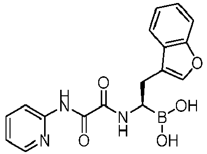

According to the method of step B and step C of example 1, 2-aminopyridine, diisopropyl ethylamine, oxalyl chloride and 2-(benzofuran-3-yl)-1-(*R*)-aminoethylboronic acid *(1S,2S,3R, 5S*)-(+)-pinane-2,3-diol ester hydrochloride were used as the starting material to obtain the product (18 mg).

¹H NMR (400 MHz, CD₃OD) δ 8.32 (d, *J* = 4.8 Hz, 1H), 8.15 (d, *J* = 8.0 Hz, 1H), 7.83 (td, *J* = 8.4 Hz, 1.6 Hz, 1H), 7.61 (d, *J* = 7.6 Hz, 1H), 7.56 (s, 1H), 7.41 (d, *J* = 8.0 Hz, 1H), 7.16-7.26 (m, 3H), 3.64 (t, *J* = 7.2 Hz, 1H), 3.09 (dd, *J* = 14.4 Hz, 6.8 Hz, 1H), 3.00 (dd, *J* = 14.4 Hz, 7.6 Hz, 1H).

### Example 13: (R)-(2-(benzofuran-3-yl)-1-(2-oxo-2-(thiazol-2-ylamino)acetamido)ethyl) boronic acid

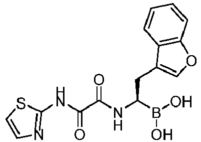

According to the method of step B and step C of example 1, 2-aminothiazole, diisopropyl ethylamine, oxalyl chloride and 2-(benzofuran-3-yl)-1-(*R*)-aminoethylboronic acid *(1S,2S,3R, 5S*)-(+)-pinane-2,3-diol ester hydrochloride were used as the starting material to obtain the product (10 mg).

¹H NMR (400 MHz, CD₃OD) δ 7.68-7.75 (m, 1H), 7.60 (d, *J* = 7.2 Hz, 1H), 7.55 (s, 1H), 7.51 (d, *J* = 3.2 Hz, 1H), 7.40 (d, *J* = 8.0 Hz, 1H), 7.16-7.25 (m, 2H), 3.67-3.70 (m, 1H), 3.08 (dd, *J* = 14.4 Hz, 6.4 Hz, 1H), 3.01 (dd, *J* = 14.8 Hz, 8.0 Hz, 1H).

### Example 14: (R)-(2-(benzofuran-3-yl)-1-(2-oxopropanamido)ethyl)boronic acid

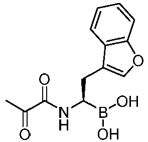

### Step A: (R)-(2-(benzofuran-3-yl)-1-(2-oxopropanamido)ethyl)boronic acid (1S,2S,3R,5S)- (+)-pinane-2,3-diol ester

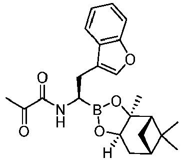

To a solution of pyruvic acid (100 mg) in tetrahydrofuran (10 mL) was added thionyl chloride (288 mg), and the mixture was heated and refluxed for 2 hours. The mixture was cooled to room temperature and concentrated under reduced pressure, the residue was dissolved in dicholoromethane (10 mL) and cooled to 0 °C, to the solution were sequentially added dropwise a solution of 2-(benzofuran-3-yl)-1-(*R*)-aminoethylboronic acid *(1S,2S,3R,5S)*-(+)-pinane-2,3- diol ester hydrochloride (450 mg) in dicholoromethane (10 mL) and diisopropylethylamine (620 mg), and the mixture was stirred at room temperature for 4 hours. 1 mol/L hydrochloric acid (5 mL) was added to quench the reaction, the organic phase was separated, washed with brine, dried over anhydrous sodium sulphate, and filtered, the filtrate was concentrated under reduced pressure, and the residue was purified through silica gel Preparative Thin-Layer Chromatography (1:1 ethyl acetate/petroleum ether) to obtain the product (110 mg).

¹H NMR (400 MHz, CDCl₃) δ 7.56 (d, *J* = 7.6 Hz, 1H), 7.43-7.46 (m, 2H), 7.19-7.28 (m, 2H), 4.28-4.32 (m, 1H), 4.36 (d, *J* = 5.2 Hz, 1H), 3.67-3.72 (m, 1H), 3.12 (dd, *J* = 14.8 Hz, 5.6 Hz, 1H), 3.00 (dd, *J* = 14.8 Hz, 7.2 Hz, 1H), 2.45 (s, 3H), 2.28-2.35 (m, 2H), 2.09-2.15 (m, 1H), 1.97-2.04 (m, 2H), 1.54-1.66 (m, 2H), 1.25-1.34 (m, 5H), 0.81 (s, 3H).

### step B: (R)-(2-(benzofuran-3-yl)-1-(2-oxopropanamido)ethyl)boronic acid

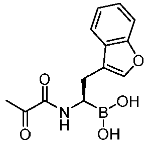

According to the method of step C of example 1, (*R*)-(2-(benzofuran-3-yl)-1-(2-oxo propanamido)ethyl)boronic acid *(1S,2S,3R,5S)*-(+)-pinane-2,3-diol ester and isobutylboronic acid were used as the starting material to obtain the product (26 mg).

¹H NMR (400 MHz, CD₃OD) δ 7.57 (d, *J* = 7.6 Hz, 1H), 7.51 (s, 1H), 7.42 (d, *J* = 8.0 Hz, 1H), 7.17-7.27 (m, 2H), 3.33-3.36 (m, 1H), 3.01 (dd, *J* = 14.4 Hz, 7.6 Hz, 1H), 2.92 (dd, *J* = 14.4 Hz, 7.6 Hz, 1H), 1.89 (s, 3H).

### Example 15: (R)-(2-(benzofuran-3-yl)-1-(2-cyclopropyl-2-oxoacetamido)ethyl)boronic acid

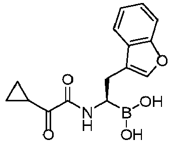

According to the method of example 14, 2-cyclopropyl-2-oxoacetic acid, thionyl chloride and 2-(benzofuran-3-yl)-1-(*R*)-aminoethylboronic acid *(1S,2S,3R,5S)*-(+)-pinane-2,3-diol ester hydrochloride were used as the main starting material to obtain the product (22 mg).

¹H NMR (400 MHz, CD₃OD) δ 7.58 (d, *J* = 7.6 Hz, 1H), 7.52 (s, 1H), 7.41 (d, *J* = 8.4 Hz, 1H), 7.18-7.27 (m, 2H), 3.44-3.48 (m, 1H), 3.01 (dd, *J* = 14.4 Hz, 7.6 Hz, 1H), 2.93 (dd, *J* = 14.8 Hz, 7.6 Hz, 1H), 2.81-2.84 (m, 1H), 1.07-1.17 (m, 4H).

### Example 16: ((R)-2-(benzofuran-3-yl)-1-(2-(4-((4-(2-((S)-2,6-dioxopiperidin-3-yl)-1,3-dioxoisoindolin-5 -yl)piperazin-1-yl)methyl)pipendin-1-yl)-2-oxoacetamido)ethyl)boronic acid

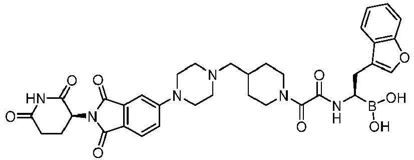

According to the method of step B and step C of example 1, (*S*)-2-(2,6-dioxopiperidin-3-yl)-5-(4-(piperidin-4-ylmethyl)piperazin-1-yl)isoindolin-1,3-dione (synthesized according to the method in patent WO2018140809A1), diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (19 mg).

¹H NMR (400 MHz, CD₃OD) δ 7.74 (d, *J* = 8.4 Hz, 1H), 7.62 (d, *J* = 7.6 Hz, 1H), 7.60 (d, *J=* 7.2 Hz, 1H), 7.46 (s, 1H), 7.42 (d, *J* = 7.6 Hz, 1H), 7.34 (d, *J* = 8.0 Hz, 1H), 7.20-7.28 (m, 2H), 5.05-5.10 (m, 1H), 4.39-4.43 (m, 1H), 4.13-4.16 (m, 2H), 3.67-3.76 (m, 3H), 3.44-3.48 (m, 4H), 3.09-3.18 (m, 2H), 2.90-3.05 (m, 2H), 2.85-2.93 (m, 1H), 2.63-2.83 (m, 4H), 2.39 (t, *J* = 8.0 Hz, 2H), 2.17-2.37 (m, 1H), 1.92-2.13 (m, 3H), 1.77-1.85 (m, 1H).

### Example 17: (R)-(2-(benzofuran-3-yl)-1-(2-oxo-2-(pyrazin-2-ylamino)acetamido)ethyl) boronic acid (1S,2S,3R,5S)-(+)-pinane-2,3-diol ester

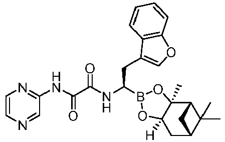

At 0 °C, to a solution of oxalyl chloride (2.5 g) in dicholoromethane (50 mL) were sequentially added dropwise a solution of 2-(benzofuran-3-yl)-1-(*R*)-aminoethylboronic acid *(1S,2S,3R,5S)*-(+)-pinane-2,3-diol ester hydrochloride (obtained according to the method of patent WO 2019/038250 A1) (1.5 g) in dicholoromethane (50 mL) and diisopropylethylamine (1.55 g), the solution was let warm up to room temperature, stirred for 2 hours, and concentrated under reduced pressure, the residue was dissolved in dicholoromethane (10 mL), to the solution was added dropwise a solution of aminopyrazine (0.38 g) in dicholoromethane (25 mL), the solution was stirred at room temperature for 4 hours, 1 mol/L hydrochloric acid (25 mL) was added to quench the reaction, the organic phase was separated, washed with brine, dried over anhydrous sodium sulphate, and filtered, the filtrate was reduced under reduced pressure, and the residue was purified through silica gel Preparative Thin-Layer Chromatography (1:1ethyl acetate/petroleum ether) to obtain the product (1.22 g).

¹H NMR (400 MHz, CDCl₃) δ 9.69 (s, 1H), 9.51 (s, 1H), 8.41 (s, 1H), 8.33 (s, 1H), 7.67 (d, *J* = 6.8 Hz, 1H), 7.58 (d, *J* = 7.2 Hz, 1H), 7.48 (s, 1H), 7.46 (d, *J* = 8.0 Hz, 1H), 7.21-7.30 (m, 2H), 4.31 (dd, *J* = 8.8 Hz, 1.6 Hz, 1H), 3.84 (dd, *J* = 13.2 Hz, 7.2 Hz, 1H), 3.18 (dd, *J* = 14.8 Hz, 5.6 Hz, 1H), 3.06 (dd, *J* = 14.8 Hz, 7.2 Hz, 1H), 2.30-2.36 (m, 1H), 2.10-2.15 (m, 1H), 1.98-2.01 (m, 1H), 1.82-1.91 (m, 2H), 1.26 (s, 3H), 1.24 (s, 3H), 1.04 (d, *J* = 10.8 Hz, 1H), 0.81 (s, 3H).

### Example 18: (R)-2,2'-(2-(2-(benzofuran-3-yl)-1-(2-oxo-2-(pyrazin-2-ylamino)acetamido) ethyl)-5-oxo-1,3,2-dioxaborolane-4,4-diyl)diacetic acid

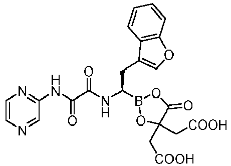

### Step A: (R)-(2-(benzofuran-3-yl)-1-(2-oxo-2-(pyrazin-2-ylamino)acetamido)ethyl)boronic acid

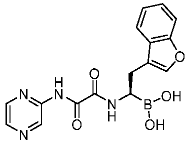

(*R*)-(2-(benzofuran-3-yl)-1-(2-oxo-2-(pyrazin-2-ylamino)acetamido)ethyl)boronic acid *(1S,2S,3R,5S)*-(+)-pinane-2,3-diol ester (1.20 g) obtained in example 1 was dissolved in methanol (50 mL), to this solution were added isobutylboronic acid (1.23 g), 1 mol/L hydrochloric acid (0.5 mL) and n-hexane (50 mL), and the mixture was stirred at room temperature overnight. The methanol phase was separated, washed with n-hexane, and concentrated under reduced pressure, and the residue was recrystallized from dicholoromethane/ethyl ether to obtain the product (835 mg).

¹H NMR (400 MHz, CD₃OD) δ 9.43 (s, 1H), 8.69 (s, 1H), 8.48 (s, 1H), 7.59 (d, *J* = 7.6 Hz, 1H), 7.54 (s, 1H), 7.38 (d, *J* = 8.0 Hz, 1H), 7.15-7.24 (m, 2H), 3.64-3.67 (m, 1H), 3.08 (dd, *J* = 14.4 Hz, 6.4 Hz, 1H), 2.99 (dd, *J* = 14.4 Hz, 8.0 Hz, 1H).

### step B: (R)-2,2'-(2-(2-(benzofuran-3-yl)-1-(2-oxo-2-(pyrazin-2-ylamino)acetamido) ethyl)-5-oxo-1,3,2-dioxaborolane-4,4-diyl)diacetic acid

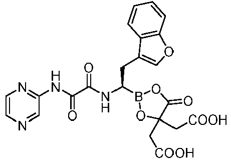

At 80 °C, to anhydrous acetonitrile (6 mL) was added anhydrous citric Acid (0.076 g), after 10 minutes (*R*)-(2-(benzofuran-3-yl)-1-(2-oxo-2-(pyrazin-2-ylamino)acetamido)ethyl)boronic acid (0.14 g) was added, the mixture was stirred for 6 hours, cooled to room temperature, and filtered, and the solid was collected to obtain the product (0.16 g).

¹H NMR (400 MHz, DMSO-*_{d6}*) δ 10.58-10.74 (brs, 1H), 9.17 (s, 1H), 8.44-8.47 (m, 2H), 7.63-7.67 (m, 2H), 7.46 (d, *J* = 8.0 Hz, 1H), 7.17-7.25 (m, 2H), 3.34-3.43 (m, 1H), 2.91 (dd, *J* = 15.2 Hz, 4.4 Hz, 1H), 2.62-2.74 (m, 3H), 2.51-2.59 (m, 2H).

### Example 19: N-((1R)-2-(1-benzofuran-3-yl)-1-(tetramethyl-1,3,2-dioxaborolan-2-yl) ethyl)-N'-(pyrazin-2-yl)oxalamide

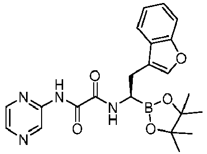

At 80 °C, to anhydrous acetonitrile (2 mL) was added pinacol (9 mg), after 10 minutes (*R*)-(2-(benzofuran-3-yl)-1-(2-oxo-2-(pyrazin-2-ylamino)acetamido)ethyl)boronic acid (26 mg) was added, and the mixture was stirred for 16 hours, cooled to room temperature, and concentrated under reduced pressure to obtain the product (30 mg).

¹H NMR (400 MHz, DMSO-*_{d6}*) δ 10.52 (s, 1H), 9.22 (d, *J* = 6.8 Hz, 1H), 9.17 (s, 1H), 8.44-8.47 (m, 2H), 7.74 (s, 1H), 7.62 (d, *J* = 7.6 Hz, 1H), 7.49 (d, *J* = 8.4 Hz, 1H), 7.25 (t, *J* = 8.0 Hz, 1H), 7.19 (t, *J* = 7.2 Hz, 1H), 3.35-3.41 (m, 1H), 2.99 (dd, *J* = 15.2 Hz, 9.2 Hz, 1H), 2.92 (dd, *J* = 15.2 Hz, 6.0 Hz, 1H), 1.17 (s, 6H), 1.16 (s, 6H).

### Example 20: N'-((1R)-2-(1-benzofiiran-3-yl)-1-(4-oxo-2,4-dihydro-1,3,2-benzo[d][1,3,2] dioxaborinin-2-yl)ethyl)-N-(pyrazin-2-yl)oxalamide

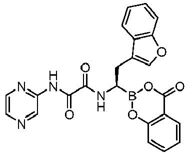

At 85 °C, to anhydrous acetonitrile (3 mL) was added salicylic acid (17 mg), after 10 minutes (R)-(2-(benzofuran-3-yl)-1-(2-oxo-2-(pyrazin-2-ylamino)acetamido)ethyl)boronic acid (44 mg) was added, and the mixture was stirred for 16 hours, cooled to room temperature, and concentrated under reduced pressure to obtain the product (50 mg).

¹H NMR (400 MHz, DMSO-*_{d6}*) δ 10.88 (s, 1H), 9.28-9.47 (brs, 1H), 9.17 (s, 1H), 8.44-8.46 (m, 2H), 7.75 (dd, *J* = 8.0 Hz, 2.0 Hz, 1H), 7.62-7.65 (m, 2H), 7.45-7.50 (m, 2H), 7.18-7.26 (m, 2H), 6.91-6.96 (m, 2H), 3.51 (q, *J* = 7.6 Hz, 1H), 2.90 (d, *J* = 7.6 Hz, 2H).

### Example 21: (R)-(2-(benzofuran-3-yl)-1-(2-(pyridin-3-yl)amino-2-oxoacetamido)ethyl) boronic acid

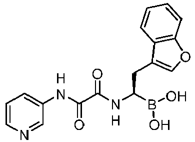

According to the method of step A of example 1 and example 2, 3-aminopyridine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (55 mg).

¹H NMR (400 MHz, CD₃OD) δ 9.42 (s, 1H), 8.77-8.80 (m, 1H), 8.61 (d, *J* = 6.0 Hz, 1H), 8.05 (dd, *J* = 8.8 Hz, 6.0 Hz, 1H), 7.63 (d, *J* = 7.2 Hz, 1H), 7.57 (s, 1H), 7.42 (d, *J* = 7.6 Hz, 1H), 7.18-7.28 (m, 2H), 3.68 (t, *J* = 7.2 Hz, 1H), 2.98-3.12 (m, 2H).

### Example 22: (R)-(2-(benzofuran-3-yl)-1-(2-(((tetrahydrofuran-2-yl)methyl)amino)-2-oxo acetamido)ethyl)boronic acid

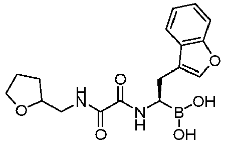

According to the method of step A of example 1 and example 2, 3-aminopyridine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (23 mg).

¹H NMR (400 MHz, CD₃OD) δ 7.59 (d, *J* = 7.6 Hz, 1H), 7.54 (s, 1H), 7.41 (d, *J* = 7.6 Hz, 1H), 7.26 (td, *J* = 7.6 Hz, 1.2 Hz, 1H), 7.20 (t, *J* = 7.6 Hz, 1H), 3.97-4.03 (m, 1H), 3.82-3.87 (m, 1H), 3.70-3.75 (m, 1H), 3.53 (t, *J* = 7.2 Hz, 1H), 3.26-3.38 (m, 2H), 2.92-3.04 (m, 2H), 1.84-2.02 (m, 3H), 1.52-1.62 (m, 1H).

### Example 23: (R)-(2-(benzofuran-3-yl)-1-(2-oxo-2-(pyridin-2-ylamino)acetamido)ethyl) boronic acid (1S,2S,3R,5S)-(+)-pinane-2,3-diol ester

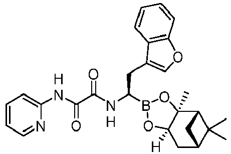

According to the method of example 1, 2-aminopyridine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (12 mg).

¹H NMR (400 MHz, CDCl₃) δ 9.70 (s, 1H), 8.35 (dd, *J* = 5.2 Hz, 1.2 Hz, 1H), 8.17 (d, *J* = 8.4 Hz, 1H), 7.73 (t, *J* = 8.0 Hz, 1H), 7.67 (d, *J* = 6.8 Hz, 1H), 7.58 (d, *J* = 7.6 Hz, 1H), 7.48 (s, 1H), 7.45 (d, *J* = 8.0 Hz, 1H), 7.20-7.30 (m, 2H), 7.08-7.11 (m, 1H), 4.31 (dd, *J* = 8.8 Hz, 1.6 Hz, 1H), 3.79 (dd, *J* = 13.2 Hz, 5.6 Hz, 1H), 3.18 (dd, *J* = 14.8 Hz, 7.6 Hz, 1H), 3.06 (dd, *J* = 14.8 Hz, 7.2 Hz, 1H), 2.29-2.36 (m, 1H), 2.09-2.16 (m, 1H), 1.98-2.00 (m, 1H), 1.85-1.89 (m, *2H), 1.26* (s, 3H), 1.24 (s, 3H), 1.06 (d, *J* = 10.8 Hz, 1H), 0.81 (s, 3H).

### Example 24: ((R)-2-(benzofuran-3-yl)-1-(2-oxo-2-((2,5-dichlorophenyl)amino)acetamido) ethyl)boronic acid

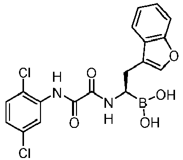

According to the method of step A of example 1 and example 2, 2,5-dichloroaniline, diisopropylethylamine, oxalyl chloride and 2-(benzofuran-3-yl)-1-(R)-aminoethylboronic acid *(1S,2S,3R,5S)*-(+)-pinane-2,3-diol ester hydrochloride were used as the starting material to obtain the product (8 mg).

¹H NMR (400 MHz, CD₃OD) δ 8.35 (d, *J* = 2.0 Hz, 1H), 7.63 (d, *J* = 7.6 Hz, 1H), 7.57 (s, 1H), 7.47 (d, *J* = 8.8 Hz, 1H), 7.43 (d, *J* = 8.0 Hz, 1H), 7.18-7.28 (m, 3H), 3.65 (t, *J* = 7.2 Hz, 1H), 2.98-3.10 (m, 2H).

### Example 25: (R)-(2-(benzofuran-3-yl)-1-(2-oxo-2-(thiazol-2-ylamino)acetamido)ethyl) boronic acid

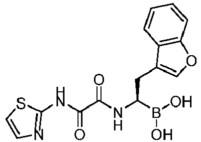

According to the method of step A of example 1 and example 2, 2-aminothiazole, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (10 mg).

¹H NMR (400 MHz, CD₃OD) δ 7.68-7.75 (m, 1H), 7.60 (d, *J* = 7.2 Hz, 1H), 7.55 (s, 1H), 7.51 (d, *J* = 3.2 Hz, 1H), 7.40 (d, *J* = 8.0 Hz, 1H), 7.16-7.25 (m, 2H), 3.67-3.70 (m, 1H), 3.08 (dd, *J* = 14.4 Hz, 6.4 Hz, 1H), 3.01 (dd, *J* = 14.8 Hz, 8.0 Hz, 1H).

### Example 26: (R)-(2-(benzofuran-3-yl)-1-(2-oxo-2-((6-methoxybenzo[d]thiazol-2-yl)amino) acetamido)ethyl)boronic acid

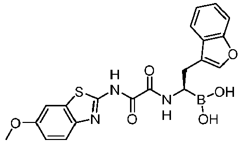

According to the method of step A of example 1 and example 2, 6-methoxybenzo[*d*] thiazol-2-amine, diisopropylethylamine, oxalyl chloride and 2-(benzofuran-3-yl)-1-(*R*)-amino ethylboronic acid *(1S,2S,3R,5.S)*-(+)-pinanc-2.3-diol ester hydrochloride were used as the starting material to obtain the product (11 mg).

¹H NMR (400 MHz, CD₃OD) δ 7.67 (d, *J* = 9.2 Hz, 1H), 7.62 (d, *J* = 7.2 Hz, 1H), 7.57 (s, 1H), 7.37-7.46 (m, 2H), 7.18-7.27 (m, 2H), 7.05 (dd, *J* = 9.2 Hz, 2.0 Hz, 1H), 3.84 (s, 3H), 3.65 (t, *J* = 7.2 Hz, 1H), 2.98-3.10 (m, 2H).

### Example 27: (R)-(2-(benzofuran-3-yl)-1-(2-oxo-2-((benzo[d]thiazol-2-yl)amino)acetamido) ethyl)boronic acid

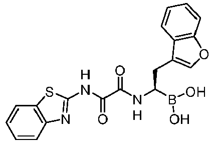

According to the method of step A of example 1 and example 2, benzo[*d*]thiazol-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (16 mg).

¹H NMR (400 MHz, CD₃OD) δ 7.89 (d, *J* = 7.6 Hz, 1H), 7.79 (d, *J* = 8.0 Hz, 1H), 7.63 (d, *J* = 7.6 Hz, 1H), 7.58 (s, 1H), 7.41-7.47 (m, 2H), 7.34 (t, *J* = 7.6 Hz, 1H), 7.18-7.27 (m, 2H), 3.66 (t, *J* = 7.6 Hz, 1H), 2.98-3.10 (m, 2H).

### Example 28: (R)-(2-(benzofuran-3-yl)-1-(2-oxo-2-((2,3-dihydrobenzofuran-7-yl)amino) acetamido)ethyl)boronic acid

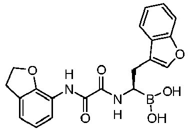

According to the method of step A of example 1 and example 2, 2,3-dihydrobenzofuran-7-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (15 mg).

¹H NMR (400 MHz, CD₃OD) δ 7.79 (d, *J* = 8.0 Hz, 1H), 7.62 (d, *J* = 8.0 Hz, 1H), 7.56 (s, 1H), 7.42 (d, *J* = 7.6 Hz, 1H), 7.19-7.27 (m, 2H), 7.03 (d, *J* = 7.2 Hz, 1H), 6.81 (t, *J* = 8.0 Hz, 1H), 4.63 (t, *J* = 8.8 Hz, 2H), 3.60 (t, *J* = 7.2 Hz, 1H), 3.25 (t, *J* = 8.8 Hz, 2H), 2.96-3.08 (m, 2H).

### Example 29: (R)-(2-(benzofuran-3-yl)-1-(2-oxo-2-(tetrahydro-1H-furo[3,4-c]pyrrol-5(3H)-yl)acetamido)ethyl)boronic acid

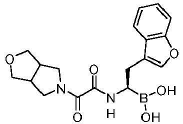

According to the method of step A of example 1 and example 2, hexahydro-1*H-*furo[3,4-*c*]pyrrole, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (8 mg).

¹H NMR (400 MHz, CD₃OD) δ 7.61 (d, *J* = 7.2 Hz, 1H), 7.57 (s, 1H), 7.42 (d, *J* = 7.6 Hz, 1H), 7.26 (td, *J* = 7.6 Hz, 1.6 Hz, 1H), 7.21 (td, *J* = 7.6 Hz, 1.2 Hz, 1H), 3.70-3.86 (m, 3H), 3.68 (dd, *J* = 12.8 Hz, 7.6 Hz, 1H), 3.61 (dd, *J* = 9.2 Hz, 3.2 Hz, 1H), 3.43-3.57 (m, 4H), 2.88-3.02 (m, 4H).

### Example 30: (R)-(2-(benzofuran-3-yl)-1-(2-oxo-2-((2,3-dihydrobenzo[b][1,4]dioxin-5-yl) amino)acetamido)ethyl)boronic acid

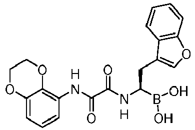

According to the method of step A of example 1 and example 2,2,3-dihydrobenzo[*b*][1,4] dioxin-5-amine, diisopropylethylamine, oxalyl chloride and 2-(benzofuran-3-yl)-1-(R)-amino ethylboronic acid *(1S,2S,3R,5S)*-(+)-pinane-2,3-diol ester hydrochloride were used as the starting material to obtain the product (6 mg).

¹H NMR (400 MHz, CD₃OD) δ 7.75 (dd, *J* = 8.4 Hz, 1.2 Hz, 1H), 7.62 (dd, *J* = 8.4 Hz, 1.2 Hz, 1H), 7.56 (s, 1H), 7.43 (d, *J* = 7.6 Hz, 1H), 7.19-7.28 (m, 2H), 6.80 (t, *J* = 7.6 Hz, 1H), 6.66 (dd, *J* = 8.4 Hz, 1.6 Hz, 1H), 4.35-4.37 (m, 2H), 4.26-4.28 (m, 2H), 3.61 (t, *J* = 7.6 Hz, 1H), 2.97-3.09 (m, 2H).

### Example 31: (R)-(2-(benzofuran-3-yl)-1-(2-oxo-2-((4-methylthiazol-2-yl)amino) acetamido)ethyl)boronic acid

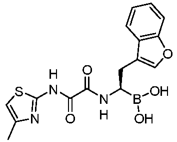

According to the method of step A of example 1 and example 2, 4-methylthiazol-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (9 mg).

¹H NMR (400 MHz, CD₃OD) δ 7.61 (d, *J* = 6.8 Hz, 1H), 7.55 (s, 1H), 7.41 (d, *J* = 8.0 Hz, 1H), 7.17-7.27 (m, 2H), 6.76 (s, 1H), 3.61-3.65 (m, 1H), 2.91-3.12 (m, 2H), 2.32 (s, 3H).

### Example 32: (R)-(2-(benzofuran-3-yl)-1-(2-oxo-2-((5-methylthiazol-2-yl)amino) acetamido)ethyl)boronic acid

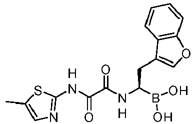

According to the method of step A of example 1 and example 2, 5-methylthiazol-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (10 mg).

¹H NMR (400 MHz, CD₃OD) δ 7.61 (d, *J* = 7.2 Hz, 1H), 7.56 (s, 1H), 7.49 (s, 1H), 7.40 (d, *J* = 8.0 Hz, 1H), 7.24 (t, *J* = 7.6 Hz, 1H), 7.19 (t, *J* = 7.6 Hz, 1H), 3.70 (t, *J* = 7.2 Hz, 1H), 2.98-3.10 (m, 2H), 2.48 (s, 3H).

### Example 33: (R)-(2-(benzofuran-3-yl)-1-(2-oxo-2-((5-fluorothiazol-2-yl)amino)acetamido) ethyl)boronic acid (1S,2S,3R,5S)-(+)-pinane-2,3-diol ester

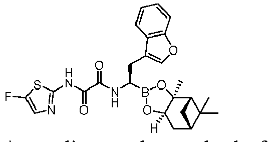

According to the method of example 1, 5-fluorothiazol-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (30 mg).

¹H NMR (400 MHz, CDCl₃) δ 7.56 (d, *J* = 7.2 Hz, 1H), 7.44-7.47 (m, 2H), 7.28 (t, *J =* 7.6 Hz, 1H), 7.22 (t, *J* = 7.6 Hz, 1H), 7.18 (d, *J* = 2.4 Hz, 1H), 4.30 (d, *J* = 8.4 Hz, 1H), 3.83-3.87 (m, 1H), 3.18 (dd, *J* = 15.2 Hz, 6.0 Hz, 1H), 3.05 (dd, *J* = 15.2 Hz, 6.8 Hz, 1H), 2.29-2.35 (m, 1H), 2.10-2.16 (m, 1H), 1.99 (t, *J* = 5.6 Hz, 1H), 1.80-1.90 (m, 2H), 1.25 (s, 3H), 1.22 (s, 3H), 1.03 (d, *J* = 11.2 Hz, 1H), 0.80 (s, 3H).

### Example 34: (R)-(2-(benzofuran-3-yl)-1-(2-oxo-2-((5-fluorothiazol-2-yl)amino)acetamido) ethyl)boronic acid

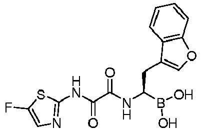

According to the method of step A of example 2, (*R*)-(2-(benzofuran-3-yl)-1-(2-oxo-2-((5-fluorothiazol-2-yl)amino)acetamido)ethyl)boronic acid *(1S,2S,3R,5S)*-(+)-pinane-2,3-diol ester and isobutylboronic acid were used as the starting material to obtain the product (6 mg).

¹H NMR (400 MHz, CD₃OD) δ 7.61 (d, *J* = 7.6 Hz, 1H), 7.55 (s, 1H), 7.41 (d, *J* = 8.4 Hz, 1H), 7.16-7.27 (m, 3H), 3.63 (t, *J* = 7.2 Hz, 1H), 2.96-3.08 (m, 2H).

### Example 35: (R)-(2-(benzofuran-3-yl)-1-(2-oxo-2-((5-chlorothiazol-2-yl)amino)acetamido) ethyl)boronic acid (1S,2S,3R,5S)-(+)-pinane-2,3-diol ester

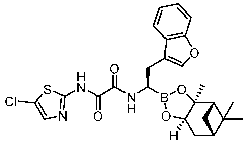

According to the method of example 1, 5-chlorothiazol-2-amine, diisopropylethyl amine, oxalyl chloride and 2-(benzofuran-3-yl)-1-(*R*)-aminoethylboronic acid *(1S,2S,3R,5S)-*(+)-pinane-2,3-diol ester hydrochloride were used as the starting material to obtain the product (25 mg).

¹H NMR (400 MHz, CDCl₃) δ 10.66-11.40 (m, 1H), 7.55-7.60 (m, 2H), 7.43-7.48 (m, 3H), 7.28 (td, *J* = 7.6 Hz, 1.2 Hz, 1H), 7.22 (td, *J* = 7.6 Hz, 1.2 Hz, 1H), 4.31 (dd, *J* = 8.8 Hz, 2.0 Hz, 1H), 3.83-3.88 (m, 1H), 3.18 (dd, *J* = 14.8 Hz, 6.0 Hz, 1H), 3.06 (dd, *J* = 14.8 Hz, 7.2 Hz, 1H), 2.28-2.36 (m, 1H), 2.10-2.16 (m, 1H), 1.99 (t, *J* = 5.6 Hz, 1H), 1.81-1.91 (m, 2H), 1.25 (s, 3H), 1.23 (s, 3H), 1.03 (d, *J* = 11.2 Hz, 1H), 0.80 (s, 1H).

### Example 36: (R)-(2-(benzofuran-3-yl)-1-(2-oxo-2-((5-chlorothiazol-2-yl)amino)acetamido) ethyl)boronic acid

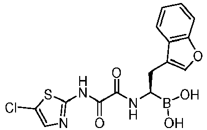

According to the method of step A of example 2, (*R*)-(2-(benzofuran-3-yl)-1-(2-oxo-2-((5-chlorothiazol-2-yl)amino)acetamido)ethyl)boronic acid *(1S,2S,3R,5S)*-(+)-pinane-2,3-diol ester and isobutylboronic acid were used as the starting material to obtain the product (9 mg).

¹H NMR (400 MHz, CD₃OD) δ 7.56 (d, *J* = 6.8 Hz, 1H), 7.50 (s, 1H), 7.32-7.39 (m, 2H), 7.11-7.23 (m, 2H), 3.55-3.63 (m, 1H), 2.90-3.05 (m, 2H).

### Example 37: (R)-(2-(benzofuran-3-yl)-1-(2-oxo-2-((1-methyl-1H-imidazol-2-yl)amino) acetamido)ethyl)boronic acid

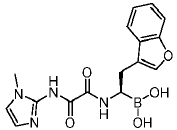

According to the method of step A of example 1 and example 2, 1-methyl-1*H-*imidazol-2-amine, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (16 mg).

¹H NMR (400 MHz, CD₃OD) δ 7.60 (d, *J* = 7.2 Hz, 1H), 7.55 (s, 1H), 7.36-7.45 (m, 3H), 7.16-7.26 (m, 2H), 3.76 (s, 3H), 3.69 (t, *J* = 6.8 Hz, 1H), 2.96-3.09 (m, 2H).

### Example 38: (R)-(2-(benzofuran-3-yl)-1-(2-oxo-2-(2-oxa-6-azaspiro[3 .3]heptan-6-yl) acetamido)ethyl)boronic acid

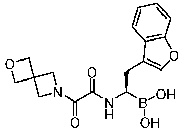

According to the method of step A of example 1 and example 2, 2-oxa-6-azaspiro[3.3] heptane, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (8 mg).

¹H NMR (400 MHz, CD₃OD) δ 7.59 (d, *J* = 7.2 Hz, 1H), 7.54 (s, 1H), 7.42 (d, *J* = 7.6 Hz, 1H), 7.19-7.28 (m, 2H), 4.23-4.33 (m, 2H), 3.82-3.88 (m, 4H), 3.72-3.73 (m, 2H), 3.42-3.44 (m, 1H), 3.00 (dd, *J* = 14.4 Hz, 7.2 Hz, 1H), 3.06 (dd, *J* = 14.4 Hz, 7.6 Hz, 1H).

### Example 39: (R)-(2-(benzofuran-3-yl)-1-(2-oxo-2-(3-oxa-8-azabicyclo[3.2.1]octan-8-yl) acetamido)ethyl)boronic acid

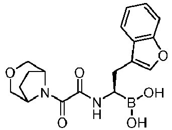

According to the method of step A of example 1 and example 2, 3-oxa-8-azabicyclo[3.2.1] octane, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (13 mg).

¹H NMR (400 MHz, CD₃OD) δ 7.60 (d, *J* = 7.6 Hz, 1H), 7.56 (s, 1H), 7.41 (d, *J* = 8.0 Hz, 1H), 7.19-7.27 (m, 2H), 4.44-4.54 (m, 2H), 3.49-3.61 (m, 5H), 2.93-2.98 (m, 2H), 1.85-1.98 (m, 4H).

### Example 40: (R)-(2-(benzofuran-3-yl)-1-(2-oxo-2-(8-oxa-3-azabicyclo[3.2.1]octan-3-yl) acetamido)ethyl)boronic acid

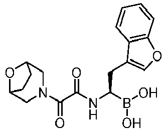

According to the method of step A of example 1 and example 2, 8-oxa-3-azabicyclo [3.2.1]octane, diisopropylethylamine, oxalyl chloride and 2-(benzofuran-3-yl)-1-(*R*)-amino ethylboronic acid *(1S,2S,3R,5S)*-(+)-pinane-2,3-diol ester hydrochloride were used as the starting material to obtain the product (10 mg).

¹H NMR (400 MHz, CD₃OD) δ 7.62 (d, *J* = 7.2 Hz, 1H), 7.58 (d, *J* = 4.4 Hz, 1H), 7.43 (d, *J* = 8.0 Hz, 1H), 7.20-7.29 (m, 2H), 4.43 (d, *J* = 6.0 Hz, 1H), 3.97 (d, *J* = 12.8 Hz, 1H), 3.51-3.60 (m, 2H), 3.18-3.29 (m, 2H), 2.88-3.01 (m, 3H), 1.65-1.86 (m, 4H).

### Example 41: (R)-(2-(benzofuran-3-yl)-1-(2-oxo-2-(2-oxa-6-azaspiro[3.4]octan-6-yl)acet amido)ethyl)boronic acid

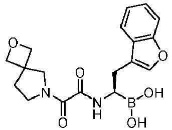

According to the method of step A of example 1 and example 2, 2-oxa-6-azaspiro[3.4] octane, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (8 mg).

¹H NMR (400 MHz, CD₃OD) δ 7.59 (d, *J* = 7.2 Hz, 1H), 7.55 (s, 1H), 7.41 (d, *J* = 8.0 Hz, 1H), 7.18-7.27 (m, 2H), 3.29-3.83 (m, 9H), 2.88-3.00 (m, 2H), 1.26-1.36 (m, 2H).

### Example 42: (R)-(2-(benzofuran-3-yl)-1-(2-oxo-2-(8-oxa-2-azaspiro[4.5]decan-2-yl)acet amido)ethyl)boronic acid

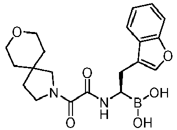

According to the method of step A of example 1 and example 2, 8-oxa-2-azaspiro[4.5] decane, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (5 mg).

¹H NMR (400 MHz, CD₃OD) δ 7.61 (d, *J* = 7.2 Hz, 1H), 7.56 (s, 1H), 7.42 (d, *J* = 8.0 Hz, 1H), 7.18-7.28 (m, 2H), 3.43-3.73 (m, 9H), 2.88-2.98 (m, 2H), 1.60-1.86 (m, 2H), 1.55-1.57 (m, 2H), 1.35-1.38 (m, 2H).

### Example 43: (R)-(2-(benzofuran-3-yl)-1-(2-oxo-2-(2-oxa-8-azaspiro[4.5]decan-8-yl)acet amido)ethyl)boronic acid

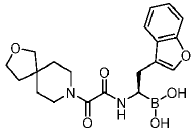

According to the method of step A of example 1 and example 2, 2-oxa-8-azaspiro[4.5] decane, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (6 mg).

¹H NMR (400 MHz, CD₃OD) δ 7.61 (d, *J* = 7.6 Hz, 1H), 7.58 (s, 1H), 7.42 (d, *J* = 8.0 Hz, 1H), 7.18-7.28 (m, 2H), 3.83-3.86 (m, 2H), 3.39-3.65 (m, 5H), 2.88-3.01 (m, 2H), 1.74-1.86 (m, 2H), 1.41-1.65 (m, 3H), 1.22-1.38 (m, 3H).

### Example 44: ((R)-2-(benzofuran-3-yl)-1-(2-oxo-2(((R)-tetrahydrofuran-3-yl)amino)acet amido)ethyl) boronic acid

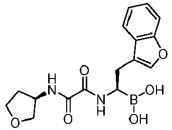

According to the method of step A of example 1 and example 2, (*R*)-3-aminotetrahydro furan p-toluenesulphonic acid salt, diisopropylethylamine, oxalyl chloride and 2-(benzofuran-3- yl)-1-(*R*)-aminoethylboronic acid *(1S,2S,3R,5S)*-(+)-pinane-2,3-diol ester hydrochloride were used as the starting material to obtain the product (12 mg).

¹H NMR (400 MHz, CD₃OD) δ 7.58 (d, *J* = 7.2 Hz, 1H), 7.53 (s, 1H), 7.41 (d, *J* = 8.4 Hz, 1H), 7.25 (t, *J* = 7.6 Hz, 1H), 7.19 (t, *J* = 7.2 Hz, 1H), 4.35-4.41 (m, 1H), 3.88-3.94 (m, 1H), 3.85 (dd, *J* = 8.8 Hz, 6.0 Hz, 1H), 3.74-3.80 (m, 1H), 3.62 (dd, *J* = 8.8 Hz, 3.6 Hz, 1H), 3.52 (t, *J* = 7.2 Hz, 1H), 3.01 (dd, *J* = 14.8 Hz, 7.2 Hz, 1H), 2.93 (dd, *J* = 14.8 Hz, 4.2 Hz, 1H), 2.17-2.26 (m, 1H), 1.87-1.94 (m, 1H).

### Example 45: (R)-(2-(benzofuran-3-yl)-1-(2-oxo-2-((2-methoxypyridin-3-yl)amino)acet amido)ethyl)boronic acid

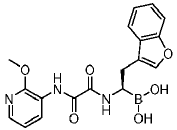

According to the method of step A of example 1 and example 2, 2-methoxypyridin-3-amine, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (21 mg).

¹H NMR (400 MHz, CD₃OD) δ 8.53 (d, *J* = 7.6 Hz, 1H), 7.69 (d, *J* = 6.8 Hz, 1H), 7.59 (d, *J* = 7.6 Hz, 1H), 7.56 (s, 1H), 7.42 (d, *J* = 7.6 Hz, 1H), 7.19-7.28 (m, 2H), 6.98 (dd, *J=* 8.0 Hz, 1.2 Hz, 1H), 4.03 (s, 3H), 3.78-3.81 (m, 1H), 2.94-3.01 (m, 2H).

### Example 46: (R)-(2-(benzofuran-3-yl)-1-(2-oxo-2-((5-methylpyrazin-2-yl)amino)acet amido)ethyl)boronic acid

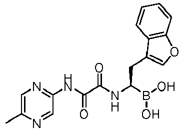

According to the method of step A of example 1 and example 2, 5-methylpyrazin-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (15 mg).

¹H NMR (400 MHz, CD₃OD) δ 9.32 (s, 1H), 8.82 (s, 0.6H), 8.78 (s, 0.4H), 7.61 (d, *J* = 7.6 Hz, 1H), 7.55 (s, 1H), 7.40 (d, *J* = 8.0 Hz, 1H), 7.17-7.26 (m, 2H), 3.64-3.68 (m, 1H), 3.08 (dd, *J* = 14.8 Hz, 6.8 Hz, 1H), 3.00 (dd, *J* = 14.4 Hz, 8.0 Hz, 1H), 2.70 (s, 1.8H), 2.69 (s, 1.2H).

### Example 47: (R)-(2-(benzofuran-3-yl)-1-(2-oxo-2-(quinoxalin-2-ylamino)acetamido)ethyl) boronic acid

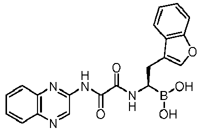

According to the method of step A of example 1 and example 2, 2-aminoquinoxaline, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (31 mg).

¹H NMR (400 MHz, CD₃OD) δ 9.66 (s, 1H), 8.05 (d, *J* = 8.0 Hz, 1H), 7.96 (d, *J* = 8.0 Hz, 1H), 7.80 (t, *J* = 7.6 Hz, 1H), 7.74 (t, *J* = 7.6 Hz, 1H), 7.63 (d, *J* = 8.0 Hz, 1H), 7.58 (s, 1H), 7.41 (d, *J* = 8.0 Hz, 1H), 7.18-7.27 (m, 2H), 3.68 (t, *J* = 7.6 Hz, 1H), 2.99-3.11 (m, 2H).

### Example 48: (R)-(2-(benzofuran-3-yl)-1-(2-oxo-2-(pyrimidin-5-ylamino)acetamido)ethyl) boronic acid

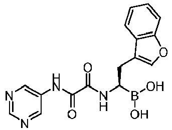

According to the method of step A of example 1 and example 2, 5-aminopyrimidine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (7 mg).

¹H NMR (400 MHz, CD₃OD) δ 9.29 (s, 2H), 9.02 (s, 1H), 7.63 (d, *J* = 7.6 Hz, 1H), 7.57 (s, 1H), 7.42 (d, *J* = 7.6 Hz, 1H), 7.26 (t, *J* = 7.6 Hz, 1H), 7.20 (t, *J* = 7.6 Hz, 1H), 3.65 (t, *J* = 7.6 Hz, 1H), 2.97-3.09 (m, 2H).

### Example 49: (R)-(2-(benzofuran-3-yl)-1-(2-oxo-2-((4-chloropyridin-2-yl)amino)acetamido) ethyl)boronic acid

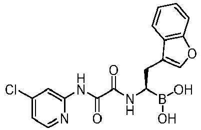

According to the method of step A of example 1 and example 2, 2-amino-4-chloro pyridine, diisopropylethylamine, oxalyl chloride and 2-(benzofuran-3-yl)-1-(*R*)-aminoethyl boronic acid *(1S,2S,3R,5S)*-(+)-pinane-2,3-diol ester hydrochloride were used as the starting material to obtain the product (14 mg).

¹H NMR (400 MHz, CD₃OD) δ 8.43 (s, 1H), 8.09 (s, 1H), 7.60-7.63 (m, 2H), 7.55 (s, 1H), 7.40 (d, *J* = 8.0 Hz, 1H), 7.24 (t, *J* = 7.6 Hz, 1H), 7.19 (t, *J* = 7.2 Hz, 1H), 3.69 (t, *J* = 7.2 Hz, 1H), 2.98-3.10 (m, 2H).

### Example 50: (R)-(2-(benzofuran-3-yl)-1-(2-oxo-2-((4-dimethylaminopyridin-2-yl)amino) acetamido)ethyl)boronic acid

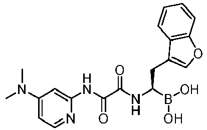

According to the method of step A of example 1 and example 2, 2-amino-4-dimethyl aminopyridine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (11 mg).

¹H NMR (400 MHz, CD₃OD) δ 7.87 (d, *J* = 5.6 Hz, 1H), 7.62 (d, *J* = 7.6 Hz, 1H), 7.56 (s, 1H), 7.41 (d, *J* = 8.0 Hz, 1H), 7.18-7.28 (m, 3H), 6.62 (d, *J* = 5.6 Hz, 1H), 3.66 (t, *J* = 7.2 Hz, 1H), 2.96-3.16 (m, 8H).

### Example 51: ((R)-1-(2-(((1S,2R,4R)-7-oxabicyclo[22.1]heptan-2-yl)amino)-2-oxoacet amido)-2-(benzofuran-3-yl)ethyl)boronic acid (1S,2S,3R,5S)-(+)-pinanc-2,3-diol ester

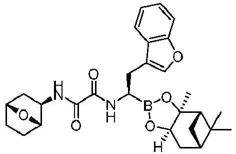

According to the method of example 1, (1*S*,2*R*,4*R*)-7-oxabicyclo[2.2.1]heptan-2-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (53 mg).

¹H NMR (400 MHz, CDCl₃) δ 7.62 (d, *J* = 6.4 Hz, 1H), 7.55 (d, *J* = 7.2 Hz, 1H), 7.52 (d, *J* = 8.8 Hz, 1H), 7.45 (s, 1H), 7.42 (d, *J* = 8.0 Hz, 1H), 7.18-7.28 (m, 2H), 4.62 (t, *J* = 9.2 Hz, 1H), 4.36 (d, *J* = 5.2 Hz, 1H), 4.27 (dd, *J* = 8.8 Hz, 1.6 Hz, 1H), 4.01 (td, *J* = 8.4 Hz, 2.8 Hz, 1H), 3.67-3.72 (m, 1H), 3.11 (dd, *J* = 14.8 Hz, 5.6 Hz, 1H), 3.00 (dd, *J* = 15.2 Hz, 7.2 Hz, 1H), 2.19-2.33 (m, 2H), 2.06-2.12 (m, 1H), 1.95-2.02 (m, 1H), 1.80-1.91 (m, 2H), 1.60-1.74 (m, 3H), 1.42-1.55 (m, 2H), 1.38 (s, 3H), 1.22-1.24 (m, 3H), 0.99 (t, *J* = 4.0 Hz, 1H), 0.78 (s, 3H).

### Example 52: (R)-(2-(benzofuran-3-yl)-1-(2-oxo-2-(2-fluorophenylamino)acetamido)ethyl) boronic acid (1S,2S,3R,5S)-(+)-pinane-2,3-diol ester

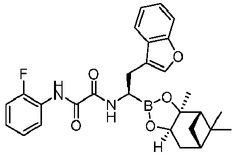

According to the method of example 1, 2-fluoroaniline, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (38 mg).

¹H NMR (400 MHz, CDCl₃) δ 9.47 (s, 1H), 8.28-8.32 (m, 1H), 7.70 (d, *J* = 6.4 Hz, 1H), 7.59 (d, *J* = 7.6 Hz, 1H), 7.49 (s, 1H), 7.45 (d, *J* = 7.6 Hz, 1H), 7.28 (t, *J* = 7.6 Hz, 1H), 7.23 (t, *J* = 7.2 Hz, 1H), 7.09-7.17 (m, 3H), 4.31 (d, *J* = 8.0 Hz, 1H), 3.82 (q, *J* = 6.8 Hz, 1H), 3.17 (dd, *J* = 14.8 Hz, 6.0 Hz, 1H), 3.06 (dd, *J* = 14.8 Hz, 7.2 Hz, 1H), 2.28-2.36 (m, 1H), 2.09-2.16 (m, 1H), 1.99 (t*, J* = 5.6 Hz, 1H), 1.81-1.91 (m, 2H), 1.25 (s, 3H), 1.24 (s, 3H), 1.03 (d, *J=* 11.2 Hz, 1H), 0.81 (s, 3H).

### Example 53: (R)-(2-(benzofuran-3-yl)-1-(2-oxo-2-((5-fluorothiazol-2-yl)amino)acetamido) ethyl)boronic acid

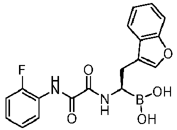

According to the method of step A of example 2, (*R*)-(2-(benzofuran-3-yl)-1-(2-oxo-2-(2- fluorophenylamino)acetamido)ethyl)boronic acid *(1S,2S,3R,5S)*-(+)-pinane-2,3-diol ester and isobutylboronic acid were used as the starting material to obtain the product (11 mg).

¹H NMR (400 MHz, CD₃OD) δ 8.02-8.06 (m, 1H), 7.63 (d, *J* = 7.6 Hz, 1H), 7.57 (s, 1H), 7.42 (d, *J* = 7.6 Hz, 1H), 7.16-7.28 (m, 5H), 3.63 (t, *J* = 7.2 Hz, 1H), 2.97-3.09 (m, 2H).

### Example 54: (R)-(2-(benzofuran-3-yl)-1-(2-oxo-2-(2,4-difluorophenylamino)acetamido) ethyl)boronic acid (1S,2S,3R,5S)-(+)-pinane-2,3-diol ester

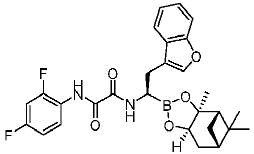

According to the method of example 1, 2,4-difluoroaniline, diisopropylethylamine, oxalyl chloride and 2-(benzofuran-3-yl)-1-*(R)*-aminoethylboronic acid *(1S,2S,3R,5S)*-(+)-pinane-2,3- diol ester hydrochloride were used as the starting material to obtain the product (40 mg).

¹H NMR (400 MHz, CDCl₃) δ 9.37 (s, 1H), 8.23-8.29 (m, 1H), 7.67 (d, *J* = 7.2 Hz, 1H), 7.59 (d, *J* = 7.6 Hz, 1H), 7.48 (s, 1H), 7.45 (d, *J* = 7.6 Hz, 1H), 7.28 (td, *J* = 7.6 Hz, 1.2 Hz, 1H), 7.22 (t, *J* = 7.2 Hz, 1H), 6.88-6.97 (m, 2H), 4.31 (dd, *J* = 8.8 Hz, 2.0 Hz, 1H), 3.82 (q, *J* = 6.8 Hz, 1H), 3.17 (dd, *J* = 14.8 Hz, 5.6 Hz, 1H), 3.06 (dd, *J* = 14.8 Hz, 7.2 Hz, 1H), 2.28-2.36 (m, 1H), 2.09-2.16 (m, 1H), 1.99 (t, *J* = 5.6 Hz, 1H), 1.81-1.90 (m, 2H), 1.25 (s, 3H), 1.24 (s, 3H), 1.02 (d, *J* = 11.2 Hz, 1H), 0.81 (s, 3H).

### Example 55: (R)-(2-(benzofuran-3-yl)-1-(2-oxo-2-(2,4-difluorophenylamino)acetamido) ethyl)boronic acid

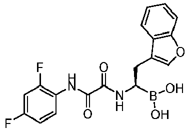

According to the method of step A of example 2, (*R*)-(2-(benzofuran-3-yl)-1-(2-oxo-2-(2,4-difluorophenylamino)acetamido)ethyl)boronic acid *(1S,2S,3R,5S)*-(+)-pinane-2,3-diol ester and isobutylboronic acid were used as the starting material to obtain the product (12 mg).

¹H NMR (400 MHz, CD₃OD) δ 7.91-7.96 (m, 1H), 7.62 (d, *J* = 7.2 Hz, 1H), 7.56 (s, 1H), 7.42 (d, *J* = 8.0 Hz, 1H), 7.26 (t, *J* = 7.2 Hz, 1H), 7.21 (t, *J* = 7.2 Hz, 1H), 7.06-7.11 (m, 1H), 6.95-7.01 (m, 1H), 3.62 (t, *J* = 7.2 Hz, 1H), 2.96-3.08 (m, 2H).

### Example 56: (R)-(2-(benzofuran-3-yl)-1-(2-oxo-2-(3,3-difluoropiperidin-1-yl)acetamido) ethyl)boronic acid

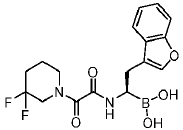

According to the method of step A of example 1 and example 2, 3,3-difluoropiperidine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (18 mg).

¹H NMR (400 MHz, CD₃OD) δ 7.60-7.63 (m, 1H), 7.56-7.57 (m, 1H), 7.42 (d, *J* = 7.6 Hz, 1H), 7.19-7.29 (m, 2H), 3.75-3.88 (m, 2H), 3.44-3.62 (m, 2H), 3.32-3.38 (m, 1H), 2.88-3.02 (m, 2H), 1.99-2.12 (m, 2H), 1.64-1.78 (m, 2H).

### Example 57: (R)-(2-(benzofuran-3-yl)-1-(2-oxo-2-((1-methyl-2-oxo-1,2-dihydropyridin-3-yl)amino)acetamido)ethyl)boronic acid (1S,2S,3R,5S)-(+)-pinane-2,3-diol ester

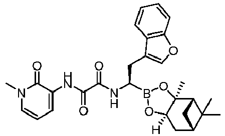

According to the method of example 1, 3-amino-1-methyl-pyridin-2(1*H*)-one, diisopropyl ethylamine, oxalyl chloride and 2-(benzofuran-3-yl)-1-(*R*)-aminoethylboronic acid *(1S,2S,3R, 5S*)-(+)-pinane-2,3-diol ester hydrochloride were used as the starting material to obtain the product (40 mg).

¹H NMR (400 MHz, CDCl₃) δ 10.70-10.85 (brs, 1H), 10.14 (s, 1H), 8.33 (dd, J = 7.6 Hz, 1.6 Hz, 1H), 7.60 (d, *J* = 6.4 Hz, 1H), 7.58 (d, *J* = 7.6 Hz, 1H), 7.47 (s, 1H), 7.44 (d, *J* = 8.0 Hz, 1H), 7.19-7.29 (m, 2H), 7.05 (dd, *J* = 6.8 Hz, 1.6 Hz, 1H), 4.30 (dd, *J* = 8.8 Hz, 1.6 Hz, 1H), 3.76 (q, *J* = 6.8 Hz, 1H), 3.64-3.79 (m, 1H), 3.61 (s, 3H), 3.16 (dd, *J* = 14.8 Hz, 5.6 Hz, 1H), 3.04 (dd, *J* = 14.8 Hz, 7.2 Hz, 1H), 2.27-2.33 (m, 1H), 2.08-2.14 (m, 1H), 1.98 (t, *J* = 5.6 Hz, 1H), 1.80-1.89 (m, 1H), 1.25 (s, 3H), 1.23 (s, 3H), 1.05 (d, *J* = 10.8 Hz, 1H), 0.80 (s, 3H).

### Example 58: (R)-(2-(benzofuran-3-yl)-1-(2-oxo-2-((1-methyl-2-oxo-1,2-dihydropyridin-3-yl)amino)acetamido)ethyl)boronic acid

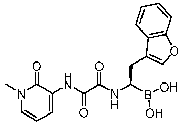

According to the method of step A of example 2, (*R*)-(2-(benzofuran-3-yl)-1-(2-oxo-2-((1-methyl-2-oxo-1,2-dihydropyridin-3 -yl)amino)acetamido)ethyl)boronic acid *(1S,2S,3R,5S)*-(+)- pinane-2,3-diol ester and isobutylboronic acid were used as the starting material to obtain the product (12 mg).

¹H NMR (400 MHz, CD₃OD) δ 8.38 (d, *J* = 7.2 Hz, 1H), 7.61 (d, *J* = 7.6 Hz, 1H), 7.55 (s, 1H), 7.41 (d, *J* = 8.0 Hz, 1H), 7.38 (d, *J* = 6.8 Hz, 1H), 7.18-7.27 (m, 2H), 6.36 (t, *J* = 6.8 Hz, 1H), 3.67-3.74 (m, 1H), 3.61 (s, 3H), 3.05 (dd, *J* = 14.0 Hz, 7.6 Hz, 1H), 2.99 (dd, *J* = 14.8 Hz, 7.6 Hz, 1H).

### Example 59: (R)-(3-methyl-1-(2-oxo-2-(thiazol-2-ylamino)acetamido)butyl)boronic acid

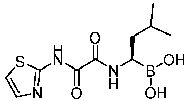

According to the method of step A of example 1 and example 2, 2-aminothiazole, diisopropylethylamine, oxalyl chloride and (*R*)-1-amino-3-methylbutylboronic acid (*1S,2S,3R, 5S*)-(+)-pinane-2,3-diol ester trifluoroacetate were used as the starting material to obtain the product (5 mg).

¹H NMR (400 MHz, CD₃OD) δ 7.80 (d, *J* = 3.2 Hz, 1H), 7.62 (d, *J* = 3.2 Hz, 1H), 3.48 (dd, *J* = 9.2 Hz, 5.2 Hz, 1H), 1.58-1.63 (m, 2H), *1.44-1.48* (m, 1H), 0.91-0.93 (m, 6H).

### Example 60: (R)-(3-methyl-1-(2-oxo-2-(pyridin-2-ylamino)acetamido)butyl)boronic acid

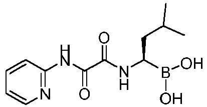

According to the method of step A of example 1 and example 2, 2-aminopyridine, diisopropylethylamine, oxalyl chloride and (*R*)-1-amino-3-methylbutylboronic acid (*1S,2S,3R, 5S*)-(+)-pinane-2,3-diol ester trifluoroacetate were used as the starting material to obtain the product (6 mg).

¹H NMR (400 MHz, CD₃OD) δ 8.46-8.51 (m, 2H), 8.02 (d, *J* = 8.8 Hz, 1H), 7.68 (t, *J* = 6.8 Hz, 1H), 3.50 (dd, *J* = 8.8 Hz, 6.0 Hz, 1H), 1.53-1.67 (m, 2H), 1.42-1.49 (m, 1H), 0.92-0.95 (m, 6H).

### Example 61: (R)-(2-(benzofuran-3-yl)-1-(2-oxo-2-methoxyacetamido)ethyl)boronic acid (1S,2S,3R,5S)-(+)-pinane-2,3-diol ester

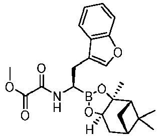

2-(benzofuran-3-yl)-1-(*R*)-aminoethylboronic acid *(1S,2S,3R,5S)*-(+)-pinane-2,3-diol ester hydrochloride(120 mg) was dissolved in dicholoromethane (20 mL), the mixture was cooled to 0 °C, diisopropylethylamine (82 mg) and methyl oxalyl chloride (40 mg) were added, the mixture was let warm up to room temperature, stirred for 1 hour, quenched with water, and extracted with dicholoromethane, the organic phase was washed with 1 mol/L hydrochloric acid and brine, dried over sodium sulphate, and filtered, the filtrate was concentrated under reduced pressure, and the residue was purified through silica gel Preparative Thin-Layer Chromatography (30%ethyl acetate/petroleum ether) to obtain the product (50 mg).

¹H NMR (400 MHz, CDCl₃) δ 7.57 (d, *J* = 6.8 Hz, 1H), 7.44-7.46 (m, 2H), 7.20-7.30 (m, 3H), 4.31 (dd, *J* = 8.8 Hz, 2.0 Hz, 1H), 3.86 (s, 3H), 3.71-3.76 (m, 1H), 3.15 (dd, *J* = 15.2 Hz, 6.0 Hz, 1H), 3.04 (dd, *J* = 15.2 Hz, 7.6 Hz, 1H), 2.28-2.35 (m, 1H), 2.09-2.16 (m, 1H), 1.99 (t, *J =* 5.6 Hz, 1H), 1.81-1.90 (m, 2H), 1.25 (s, 3H), 1.24 (s, 3H), 1.07 (d, *J* = 11.6 Hz, 1H), 0.80 (s, 3H).

### Example 62: (R)-(2-(benzofuran-3-yl)-1-(2-oxo-2-methoxyacetamido)ethyl)boronic acid

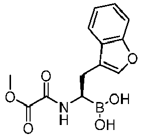

According to the method of step A of example 2, (*R*)-(2-(benzofuran-3-yl)-1-(2-oxo-2-methoxyacetamido)ethyl)boronic acid *(1S,2S,3R,5S)*-(+)-pinane-2,3-diol ester was used as the starting material to obtain the product (18 mg).

¹H NMR (400 MHz, CD₃OD) δ 7.60 (d, *J* = 8.0 Hz, 1H), 7.54 (s, 1H), 7.42 (d, *J* = 8.0 Hz, 1H), 7.26 (t, *J* = 8.0 Hz, 1H), 7.21 (t, *J* = 8.0 Hz, 1H), 3.84 (s, 3H), 3.52 (t, *J* = 7.6 Hz, 1H), 2.93-3.05 (m, 2H).

### Example 63: (R)-2-((2-(benzofuran-3-yl)-1-boronethyl)amino)-2-oxoacetic acid

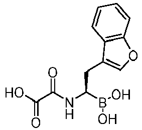

(*R*)-2-(benzofuran-3-yl)-1-(2-methoxy-2-oxoacetamido)ethyl)boronic acid *(1S,2S,3R,5S)-* (+)-pinane-2,3-diol ester (20 mg) was dissolved in methanol (2 mL) and water (2 mL), to the mixture was added lithium hydroxide monohydrate (8 mg), and the mixture was stirred at room temperature for 2 hours. To the mixture were added 1 mol/L hydrochloric acid (1 mL), isobutylboronic acid (40 mg) and n-hexane (5 mL), and the mixture was stirred at room temperature overnight. The methanol phase was separated and washed with n-hexane, then dicholoromethane (10 mL) was added, the mixture was washed with water and concentrated under reduced pressure to obtain the product (3 mg).

¹H NMR (400 MHz, CD₃OD) δ 7.60 (d, *J* = 8.0 Hz, 1H), 7.55 (s, 1H), 7.42 (d, *J* = 8.0 Hz, 1H), 7.26 (t, *J* = 7.6 Hz, 1H), 7.21 (t, *J* = 7.6 Hz, 1H), 3.44-3.50 (m, 1H), 2.91-3.05 (m, 2H).

### Example 64: (R)-(2-(benzofuran-3-yl)-1-(2-oxo-2-((2-chloropyridin-3-yl)amino)acetamido) ethyl)boronic acid

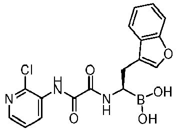

According to the method of step A of example 1 and example 2, 2-chloropyridin-3-amine, diisopropylethylamine, oxalyl chloride and 2-(benzofuran-3-yl)-1-(*R*)-aminoethylboronic acid *(1S,2S,3R,5S)*-(+)-pinane-2,3-diol ester hydrochloride were used as the starting material to obtain the product (21 mg).

¹H NMR (400 MHz, CD₃OD) δ 8.62 (dd, *J* = 8.0 Hz, 1.6 Hz, 1H), 8.17 (dd, *J* = 4.8 Hz, 1.6 Hz, 1H), 7.62 (d, *J* = 8.0 Hz, 1H), 7.56 (s, 1H), 7.40-7.44 (m, 2H), 7.25 (td, *J* = 7.6 Hz, 1.2 Hz, 1H), 7.20 (td, *J* = 7.6 Hz, 0.8 Hz, 1H), 3.65 (t, *J* = 7.6 Hz, 1H), 3.07 (dd, *J* = 14.4 Hz, 7.2 Hz, 1H), 3.01 (dd, *J* = 14.4 Hz, 7.2 Hz, 1H).

### Example 65: (R)-(2-(benzofuran-3-yl)-1-(2-oxo-2-(1,4-dioxa-8-azaspiro[4.5]decan-8-yl) acetamido)ethyl)boronic acid (1S,2S,3R,5S)-(+)-pinanc-2.3-diol ester

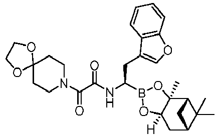

According to the method of example 1, 1,4-dioxa-8-azaspiro[4.5]decane, diisopropylethyl amine, oxalyl chloride and 2-(benzofuran-3-yl)-1-(*R*)-aminoethylboronic acid *(1S,2S,3R,5S)-* (+)-pinane-2,3-diol ester hydrochloride were used as the starting material to obtain the product (28 mg).

¹H NMR (400 MHz, CDCl₃) δ 7.58 (dd, *J* = 8.0 Hz, 1.2 Hz, 1H), 7.50 (s, 1H), 7.44 (d, *J* = 8.0 Hz, 1H), 7.24-7.31 (m, 2H), 7.22 (td, *J* = 7.2 Hz, 1H), 4.28 (dd, *J* = 9.2 Hz, 2.0 Hz, 1H), 4.00-4.03 (m, 2H), 3.96 (s, 4H), 3.67-3.70 (m, 2H), 3.58-3.63 (m, 1H), 3.12 (dd, *J* = 15.2 Hz, 5.2 Hz, 1H), 2.99 (dd, *J* = 15.2 Hz, 8.0 Hz, 1H), 2.27-2.34 (m, 1H), 2.06-2.13 (m, 1H), 1.97 (t, *J* = 5.2 Hz, 1H), 1.79-1.88 (m, 2H), 1.70-1.75 (m, 4H), 1.24 (s, 3H), 1.23 (s, 3H), 1.07 (d, *J* = 11.2 Hz, 1H), 0.80 (s, 3H).

### Example 66: (R)-(2-(benzofuran-3-yl)-1-(2-oxo-2-(1,4-dioxa-8-azaspiro[4.5]decan-8-yl) acetamido)ethyl)boronic acid

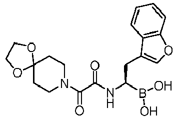

According to the method of step A of example 2, (*R*)-(2-(benzofuran-3-yl)-1-(2-oxo-2-(1,4-dioxa-8-azaspiro[4.5]decan-8-yl)acetamido)ethyl)boronic acid *(1S,2S,3R,5S)*-(+)-pinane-2,3-diol ester and isobutylboronic acid were used as the starting material to obtain the product (7 mg).

¹H NMR (400 MHz, CD₃OD) δ 7.62 (d, *J* = 7.6 Hz, 1H), 7.58 (s, 1H), 7.43 (d, *J* = 7.6 Hz, 1H), 7.20-7.28 (m, 2H), 3.94 (s, 4H), 3.49-3.63 (m, 3H), 3.36-3.39 (m, 2H), 2.89-3.02 (m, 2H), 1.65-1.68 (m, 2H), 1.57-1.60 (m, 2H).

### Example 67: ((1R)-2-(1-benzofuran-3-yl)-1-(4,4,6-trimethyl-1,3,2-dioxaborinan-2-yl) ethyl)-N'-(pyrazin-2-yl)oxalamide

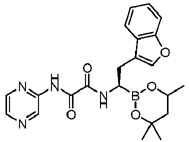

According to the method of example 19, (*R*)-(2-(benzofuran-3-yl)-1-(2-oxo-2-(pyrazin-2- ylamino)acetamido)ethyl)boronic acid and 2-methylpentane-2,4-diol were used as the starting material to obtain the product (15 mg).

¹H NMR (400 MHz, DMSO-*_{d6}*) δ 10.50 (s, 1H), 9.19 (d, *J* = 2.8 Hz, 1H), 8.80 (d, *J* = 6.8 Hz, 0.5H), 8.71 (d, *J* = 6.8 Hz, 0.5H), 8.45 (dd, *J* = 6.4 Hz, 1.2 Hz, 2H), 7.71 (s, 0.5H), 7.70 (s, 0.5H), 7.61 (d, *J* = 7.2 Hz, 1H), 7.49 (d, *J* = 8.4 Hz, 1H), 7.18-7.27 (m, 2H), 4.10-4.15 (m, 1H), 3.24-3.29 (m, 1H), 2.90-2.94 (m, 2H), 1.78-1.81 (m, 1H), 1.99-2.12 (m, 2H), 1.30-1.37 (m, 1H), 1.11-1.27 (m, 7H).

### Example 68: (1R)-2-(1-benzofuran-3-yl)-1-(4-methyl-1,3,2-dioxaborolan-2-yl)ethyl)-N'-(pyrazin-2-yl)oxalamide

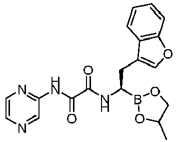

According to the method of example 19, (*R*)-(2-(benzofuran-3-yl)-1-(2-oxo-2-(pyrazin-2- ylamino)acetamido)ethyl)boronic acid and propane-1,2-diol were used as the starting material to obtain the product (53 mg).

¹H NMR (400 MHz, DMSO-*_{d6}*) δ 10.40 (s, 1H), 9.20 (s, 1H), 8.58 (d, *J* = 8.4 Hz, 1H),8.46 (d, *J* = 2.4 Hz, 1H), 8.45 (d, *J* = 4.0 Hz, 1H), 7.65-7.68 (m, 2H), 7.48 (d, *J* = 8.0 Hz, 1H), 7.18-7.27 (m, 2H), 4.37 (t, *J* = 5.6 Hz, 1H), 3.52-3.57 (m, 2H), 3.22-3.33 (m, 1H), 3.12-3.16 (m, 1H), 2.97-3.00 (m, 1H), 0.97 (d, *J* = 6.0 Hz, 3H).

### Example 69: N-(1R)-2-(1-benzofuran-3-yl)-1-((R)-4-methyl-1,3,2-dioxaborolan-2-yl) ethyl)-N'-(pyrazin-2-yl)oxalamide

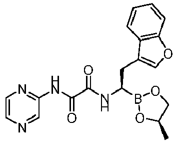

According to the method of example 19, (R)-(2-(benzofuran-3-yl)-1-(2-oxo-2-(pyrazin-2-ylamino)acetamido)ethyl)boronic acid and (R)-propane-1,2-diol were used as the starting material to obtain the product (22 mg).

¹H NMR (400 MHz, DMSO*_{-d6}*) δ 10.41 (s, 1H), 9.20 (s, 1H), 8.58 (d, *J* = 8.4 Hz, 1H), 8.46 (d, *J* = 2.4 Hz, 1H), 8.45 (d, *J* = 4.0 Hz, 1H), 7.65-7.68 (m, 2H), 7.48 (d, *J* = 8.0 Hz, 1H), 7.18-7.27 (m, 2H), 4.42 (t, *J* = 5.6 Hz, 1H), 3.52-3.57 (m, 2H), 3.21-3.32 (m, 1H), 3.12-3.16 (m, 1H), 2.97-3.00 (m, 1H), 0.98 (d, *J* = 6.4 Hz, 3H).

### Example 70: (R)-N-2-(1-benzofuran-3-yl)-1-(1,3,2-dioxaborinan-2-yl)ethyl)-N'-(pyrazin-2-yl)oxalamide

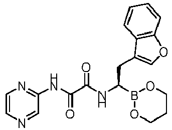

According to the method of example 19, (R)-(2-(benzofuran-3-yl)-1-(2-oxo-2-(pyrazin-2- ylamino)acetamido)ethyl)boronic acid and propane-1,3-diol were used as the starting material to obtain the product (21 mg).

¹H NMR (400 MHz, DMSO_{-*d6*}) δ 10.41 (s, 1H), 9.20 (d, *J* = 3.2 Hz, 1H), 8.71 (d, *J* = 6.8 Hz, 1H), 8.45 (dd, *J* = 6.4 Hz, 1.2 Hz, 2H), 7.69 (s, 1H), 7.61 (d, *J* = 8.0 Hz, 1H), 7.48 (d, *J* = 8.4 Hz, 1H), 7.19-7.27 (m, 2H), 4.26-4.29 (m, 1H), 3.52-3.58 (m, 4H), 2.97-3.05 (m, 2H), 1.51-1.57 (m, 2H).

### Example 71: (R)-N-2-(1-benzofuran-3-yl)-1-(5,5-dimethyl-1,3,2-dioxaborinan-2-yl)ethyl)-N'-(pyrazin-2-yl)oxalamide

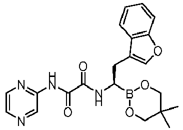

According to the method of example 19, (*R*)-(2-(benzofuran-3-yl)-1-(2-oxo-2-(pyrazin-2- ylamino)acetamido)ethyl)boronic acid and 2,2-dimethylpropane-1,3-diol were used as the starting material to obtain the product (30 mg).

¹H NMR (400 MHz, DMSO_{-*d6*}) δ 10.50 (s, 1H), 9.17 (s, 1H), 8.99 (d, *J* = 7.2 Hz, 1H), 8.45 (d, *J* = 1.2 Hz, 1H), 8.44 (d, *J* = 2.8 Hz, 1H), 7.71 (s, 1H), 7.61 (d, *J* = 7.2 Hz, 1H), 7.48 (d, *J* = 8.0 Hz, 1H), 7.17-7.26 (m, 2H), 3.56 (s, 4H), 3.34-3.39 (m, 1H), 3.30 (m, 2H), 0.87 (s, 6H).

### Example 72: (R)-N-2-(1-benzofuran-3-yl)-1-(4,4,6,6-tetramethyl-1,3,2-dioxaborinan-2-yl) ethyl)-N'-(pyrazin-2-yl)oxalamide

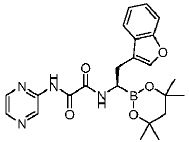

According to the method of example 19, (R)-(2-(benzofuran-3-yl)-1-(2-oxo-2-(pyrazin-2- ylamino)acetamido)ethyl)boronic acid and 2,4-dimethylpentane-2,4-diol were used as the starting material to obtain the product (53 mg).

¹H NMR (400 MHz, DMSO_{-*d6*}) δ 10.48 (s, 1H), 9.20 (d, *J* = 1.2 Hz, 1H), 8.77 (d, *J* = 6.8 Hz, 1H), 8.45-8.47 (m, 2H), 7.72 (s, 1H), 7.62 (d, *J* = 7.2 Hz, 1H), 7.49 (d, *J* = 8.4 Hz, 1H), 7.18-7.27 (m, 2H), 3.21-3.26 (m, 1H), 2.92-2.95 (m, 2H), 1.76 (s, 1H), 1.30-1.37 (m, 1H), 1.21 (s, 12H).

### Example 73: (R)-N-2-(benzofuran-3-yl)-1-(4-oxo-1,3,2-dioxaborolan-2-yl)ethyl)-N'-(pyrazin-2-yl)oxalamide

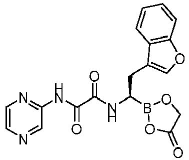

According to the method of example 20, (R)-(2-(benzofuran-3-yl)-1-(2-oxo-2-(pyrazin-2- ylamino)acetamido)ethyl)boronic acid and 2-hydroxyacetic acid were used as the starting material to obtain the product (112 mg).

¹H NMR (400 MHz, DMSO_{-*d6*}) δ 10.43 (s, 1H), 9.20 (s, 1H), 8.60 (d, *J* = 8.4 Hz, 1H), 8.42-8.46 (m, 2H), 7.62-7.68 (m, 2H), 7.47 (d, *J* = 8.4 Hz, 1H), 7.18-7.26 (m, 2H), 3.88 (s, 1H), 3.51-3.57 (m, 1H), 2.92-3.03 (m, 1H), 2.81 (d, *J* = 6.8 Hz, 2H).

### Biological test

### 1. Determination of the enzymatic inhibitory activity of the compound on LMP7:

LMP7 is a catalytic subunit of immunoproteasomes. In this experiment, an enzymatic detection platform was established using its hydrolytic enzyme activity and used for compound activity detection. Using Ac-ANW-AMC (Bonston Biochem, Cat # S-320) as a substrate for LMP7, the amount of fluorescent group AMC (7-amino-4-methylcoumarin) released after hydrolysis can reflect the enzymatic activity. MOLT-4 cells are human acute lymphoblastic leukemia cells and have been identified as cells with high expression of LMP7. We established an enzymatic detection method for compound inhibition on LMP7 using MOLT-4 cell lysate as the enzyme source of LMP7 and detected the compound inhibitory activity (half inhibitory concentration, IC₅₀).

MOLT-4 cells were cultured in a 75 cm² cell culture flask (Coming) (37°C, 95% air, and 5% CO₂) with RPMI-1640 (Biological Industries) medium containing 10% fetal bovine serum (Biological Industries) and 1% Pen Strep (Gibco) and passaged 2-3 times one week. 1 × 10⁷ MOLT-4 cells were collected, resuspended in 1 ml of PBS (Solarbio), and centrifuged at 3000 rpm for 5 minutes. The supernatant was aspirated off. The cells were resuspended in 500 µl of lysis buffer (20 mM Tris, pH 8.0, 5 mM EDTA, plus Protease inhibitor (1: 1000) and Phosphatase Inhibitor (1: 100) when used) and left on ice for 30 minutes. The cells were broken by supersonic technique, 0.5 S on, 0.5 S off, for 2.5 S. The centrifuge was performed at 12000 rpm, 4°C for 10 minutes. The supernatant was the cell lysate and protein quantification was performed by using the BCA method (Thermo, #23225).

Compounds were diluted in a 5-fold gradient with 100% DMSO for a total of 9 concentrations. 2 µl for each concentration was added to a 48-µl reaction buffer (20 mM Tris, pH 8.0, 0.5 mM EDTA) and mixed well as 4* compound (final concentrations: 2000, 400, 80, 16, 3.2, 0.64, 0.128, 0.0256, and 0 nM) for later use. A reaction buffer was used to prepare 4* MOLT-4 cell lysate at a final concentration of 20 ng/µl and 2*Ac-ANW-AMC at a final concentration of 100 µM. 5 µl of 4* compound was added to a 384-well plate (OptiPlate-384, purchased from PerkinElmer), added with 5 µl of 4* cell lysate, centrifuged, and reacted in an incubator at 23°C for 1 hour. 10 µl of 2* Ac-ANW-AMC was added and the reaction was started by centrifugation at 23°C in the dark for 2 hours. After completion of the reaction, the signal value (excitation wavelength 345 nm/emission wavelength 445 nm) was read on the CLARIO star^{Plus} (purchased from BMG LRBTECH). Enzyme activity was determined at 9 concentrations for each compound, and data were processed using the GraphPad Prism software to calculate the half inhibitory concentration of the compound on LMP7, i.e. the IC₅₀ value.

### 2. Determination of the enzymatic inhibitory activity of the compound on β5:

β5 is a catalytic subunit of immunoproteasomes. In this experiment, an enzymatic detection platform was established using its hydrolytic enzyme activity and used for compound activity detection. Using Ac-WLA-AMC (Bonston Biochem, Cat # S-330) as a substrate for β5, the amount of fluorescent group AMC (7-amino-4-methylcoumarin) released after hydrolysis can reflect the enzymatic activity. HEK-293 is a human embryonic kidney cell that constitutively expresses proteasome and does not express immunoproteasome. We established an enzymatic detection method for compound inhibition on β5 using HEK-293 cell lysate as the enzyme source of β5 and detected the compound inhibitory activity (half inhibitory concentration, IC₅₀). The enzymatic inhibitory activity of compounds against β5 was used as a measure index of compound selectivity.

HEK-293 cells were cultured in a 75 cm² cell culture flask (Coming) (37°C, 95% air, and 5% CO₂) with DMEM (Biological Industries) medium containing 10% fetal bovine serum (Biological Industries) and 1% Pen Strep (Gibco) and passaged 2-3 times one week. 1 × 10⁷ HEK-293 cells were collected, resuspended in 1 ml of PBS (Solarbio), and centrifuged at 3000 rpm for 5 minutes. The supernatant was aspirated off. The cells were resuspended in 500 µl of lysis buffer (20 mM Tris, pH 8.0, 5 mM EDTA, plus Protease inhibitor (1: 1000) and Phosphatase Inhibitor (1: 100) when used) and left on ice for 30 minutes. The cells were broken by supersonic technique, 0.5 S on, 0.5 S off, for 2.5 S. The centrifuge was performed at 12000 rpm, 4°C for 10 minutes. The supernatant was the cell lysate and protein quantification was performed using the BCA method (Thermo, #23225).

Compounds were diluted in a 5-fold gradient with 100% DMSO for a total of 9 concentrations. 2 µl for each concentration was added to a 48-µl reaction buffer (20 mM Tris, pH 8.0, 0.5 mM EDTA) and mixed well as 4* compound (final concentrations: 100000, 20000, 4000, 800, 160, 32, 6.4, 1.28, and 0 nM) for later use. A reaction buffer was used to prepare 4*HEK-293 cell lysate at a final concentration of 25 ng/µl and 2*Ac-WLA-AMC at a final concentration of 20 µM. 5 µl of 4* compound was added to a 384-well plate (OptiPlate-384, purchased from PerkinElmer), added with 5 µl of 4*HEK-293 cell lysate, centrifuged, and reacted in an incubator at 23°C for 1 hour. 10 µl of 2* Ac-WLA-AMC was added and the reaction was started by centrifugation at 23°C in the dark for 2 hours. After completion of the reaction, the signal value (excitation wavelength 345 nm/emission wavelength 445 nm) was read on the CLARIO star^{Plus} (purchased from BMG LRBTECH). Enzyme activity was determined at 9 concentrations for each compound, and data were processed using the GraphPad Prism software to calculate the half inhibitory concentration of the compound on β5, i.e. the IC₅₀ value.

### 3. Determination of the cytological inhibitory activity of the compound on LMP7:

MOLT-4 are human acute lymphoblastic leukemia cells and have been identified as cells with high expression of LMP7. In this experiment, the compounds were pre-incubated with MOLT-4, and Ac-ANW-AMC (BonstonBiochem, Cat# S-320) was used as the substrate of LMP7 to establish the detection method for the enzymatic activity of LMP7 in cells and detect the compound inhibitory activity (half inhibitory concentration, IC₅₀).

Cells were plated in a 24-well plate (Coming) at a concentration of 1.5 × 10⁵/ml per well. The next day compounds were prepared and diluted in a 5-fold gradient from 2 mM in 100% DMSO for a total of 8 concentrations. 2 µl for each concentration of the compound was added to a 1-ml cell culture medium, supplemented with 1 ml of RPMI-1640 only (final FBS concentration 5%, v/v). The compounds were mixed with the cells. After incubation for 2 hours in the incubator, the cells were centrifuged at 2000 rpm for 8 minutes to discard the supernatant. The cells were washed once with 2 ml PBS (Solarbio), and centrifuged again to discard the supernatant. The cells were resuspended in 100 µl of lysis buffer (20 mM Tris, pH 8.0, 5 mM EDTA, plus Protease inhibitor (1: 100) and Phosphatase Inhibitor (1: 100) when used) and left in a 4°C refrigerator for 45 minutes. After 2 min. of centrifuge at 2000 rpm, the lysate was transferred from the 24-well plate to a 96-well plate, and centrifuged at 2000 rpm, 4°C, for 15 min. The supernatant was the lysate. Protein quantification was performed using the BCA method (Thermo, #23225). A 2* lysate was prepared with the reaction buffer (20 mM Tris, pH 8.0, 0.5 mM EDTA) at a final concentration of 20 ng/µl. 2* Ac-ANW-AMC was prepared with the reaction buffer at a final concentration of 100 µM. 10 µl of 2* lysate was added to a 384-well plate (OptiPlate-384, purchased from PerkinElmer), and added with 10 µl of 2* Ac-ANW-AMC. The reaction was started by centrifugation at 23°C in the dark for 1 hour. After completion of the reaction, the signal value (excitation wavelength 345 nm/emission wavelength 445 nm) was read on the CLARIO starPlus (purchased from BMG LRBTECH). Enzyme activity was determined at 8 concentrations for each compound, and data were processed using the GraphPad Prism software to calculate the half inhibitory concentration of the compound on LMP7 hydrolase in MOLT-4 cells, i.e. the IC₅₀ value.

The aforementioned "*" refers to multiplication, representing multiples.

The test results of some compounds above are shown in Table 1.

**Table 1: In vitro activity test results of compounds**

| Compound | Enzymatic activity data | | Cytological activity data |
|---|---|---|---|
| | LMP7 Activity (MOLT-4 Cell Lysate, nM) | β5 Activity (HEK-293 Cell Lysate, nM) | LMP7 Activity (MOLT-4Cell, nM) |
| Ex. 1 | 7.65 | 154 | 5.94 |
| Ex. 2 | 26.9 | N.D.* | N.D. |
| Ex. 3 | 13 | 507 | N.D. |
| Ex. 4 | 22.3 | N.D. | N.D. |
| Ex. 5 | 15.6 | N.D. | N.D. |
| Ex. 6 | 28.5 | N.D. | N.D. |
| Ex. 7 | 7.7 | 123 | N.D. |
| Ex. 8 | 24.7 | N.D. | N.D. |
| Ex. 9 | 9.02 | 97.5 | 4.82 |
| Ex. 11 | 7.3 | 123 | N.D. |
| Ex. 12 | 9.64 | 283 | 3.77 |
| Ex. 13 | 3.13 | 58.7 | 4.06 |
| Ex. 16 | 32.9 | 58.7 | N.D. |
| Ex. 17 | 2.60 | 30.1 | 0.80 |
| Ex. 18 | 2.42 | 34.1 | 1.26 |
| Ex. 19 | 1.11 | 12.2 | 0.69 |
| Ex. 20 | 1.20 | 18.8 | 0.32 |
| Ex. 21 | 2.86 | 29 | 3.45 |
| Ex. 22 | 3.57 | 22.2 | 1.83 |
| Ex. 23 | 3.81 | 126 | 1.65 |
| Ex. 24 | 6.27 | 115 | 3.92 |
| Ex. 25 | 3.13 | 58.7 | 4.06 |
| Ex. 26 | 4.86 | 19.4 | 0.73 |
| Ex. 27 | 3.53 | 44.6 | 1.58 |
| Ex. 28 | 3.82 | 22.6 | 3.84 |
| Ex. 29 | 7.81 | 41.4 | 15.9 |
| Ex. 30 | 5.61 | 24.6 | 8.77 |
| Ex. 31 | 2.91 | 72.5 | 11.0 |
| Ex. 32 | 4.60 | 62.9 | 1.72 |
| Ex. 33 | 3.08 | 39.0 | 9.50 |
| Ex. 34 | 7.46 | 132 | 29.2 |
| Ex. 35 | 1.99 | 38.4 | 13.6 |
| Ex. 36 | 3.14 | 54.5 | 19.5 |
| Ex. 37 | 5.93 | 264 | 111 |
| Ex. 39 | 5.95 | 392 | 3.51 |
| Ex. 44 | 4.91 | 54.2 | 15.9 |
| Ex. 45 | 5.32 | 97.7 | 22.7 |
| Ex. 46 | 7.83 | 93.8 | 2.29 |
| Ex. 47 | 3.10 | 43.5 | 2.32 |
| Ex. 48 | 4.58 | 22.5 | 11.1 |
| Ex. 49 | 1.51 | 61.1 | 1.90 |
| Ex. 50 | 5.96 | 357 | 7.91 |
| Ex. 51 | 3.08 | 74.6 | 3.88 |
| Ex. 52 | 1.61 | 32.4 | 4.94 |
| Ex. 53 | 1.88 | 64.7 | 12.7 |
| Ex. 54 | 0.89 | 23.7 | 5.33 |
| Ex. 55 | 1.43 | 54.6 | 6.84 |
| Ex. 56 | 7.48 | 88.4 | 6.39 |
| Ex. 57 | 1.32 | 140 | 4.03 |
| Ex. 58 | 0.95 | 84.1 | 5.45 |
| Ex. 61 | 4.27 | 722 | 46.6 |
| Ex. 62 | 7.89 | 1301 | 26.7 |
| Ex. 64 | 2.31 | 25.4 | 1.79 |
| Ex. 67 | 1.59 | 38.2 | 0.79 |
| Ex. 68 | 1.24 | 34.8 | 1.10 |
| Ex. 69 | 1.44 | 18.2 | 0.88 |
| Ex. 70 | 1.50 | 26.0 | 0.91 |
| Ex. 71 | 1.18 | 28.5 | 1.35 |
| Ex. 72 | 1.46 | 21.8 | 0.98 |
| Ex. 73 | 1.23 | 20.7 | 1.15 |

| | | | |
|---|---|---|---|
| N. D.: Not tested | | | |

### 4. Animal Pharmacokinetic Studies of Compounds:

Three healthy adult male rats from Beijing Vital River Experimental animal Technology Co. Ltd were used for animal pharmacokinetic experiments. The compounds were suspended in 20% sulfobutyl ether-β-cyclodextrin (W/W/V) at a concentration of 1 mg/mL at a dosing volume of 5 mL/kg by single gavage at a dose of 5 mg/kg. Animals were fasted overnight before the experiment, from 10 hours before the administration to 4 hours after the administration. Blood was collected at 0.25, 0.5, 1, 2, 4, 6, 8, and 24 hours after the administration. The animals were anesthetized with isoflurane. About 0.4 mL of whole blood was collected from the orbital venous plexus using a glass blood collection tube and placed in a heparin anticoagulant tube. The sample was centrifuged at 4200 rpm for 5 min at 4°C. The plasma was transferred to a centrifuge tube and stored at -80°C for later analysis. The plasma sample analysis used the acetonitrile protein precipitation method to extract the compound to be tested and internal standard (Warfarin or Propranolol) from rat plasma, and the extraction solution was analyzed by LC/MS/MS. The plasma concentration-time data of individual animals measured were analyzed using a non-compartmental model using the WinNonlin (version 5.2.1; Pharsight company) software, and the following pharmacokinetic parameters were obtained: maximum (peak) plasma drug concentration Cₘₐₓ; Time to max Tₘₐₓ; the area under the curve AUC_{0-inf} of blood drug concentration-time from half-life T1/2 and extrapolation to infinite time.

| Compound | AUC_{0-inf} (hr*ng/mL) | T_{1/2} (hr) | Tₘₐₓ (hr) | Cₘₐₓ (ng/mL) |
|---|---|---|---|---|
| Ex. 5 | 20855 | 4.20 | 1.17 | 3797 |
| Ex. 9 | 38582 | 5.58 | 2.67 | 3878 |
| Ex. 12 | 26545 | 14.69 | 0.58 | 3933 |
| Ex. 25 | 108161 | 14.4 | 0.42 | 7710 |
| Ex. 27 | 24212 | 5.17 | 3.33 | 1913 |
| Ex. 32 | 22200 | 25.3 | 0.67 | 1090 |
| Ex. 49 | 25387 | 16.39 | 0.92 | 1430 |
| Ex 19* | 22190 | 8.99 | 2.67 | 1763 |
| Ex 67* | 22306 | 10.7 | 0.42 | 2420 |
| Ex 70* | 34446 | 16.5 | 0.42 | 5414 |
| Ex 71* | 24504 | 11 | 0.5 | 5284 |

| | | | | |
|---|---|---|---|---|
| *-marked compound was the prodrug of the compound of Example 9, and its animal pharmacokinetic data is measured by measuring the signal of the parent drug, i.e. the compound of Example 9. | | | | |

### 5. In-vivo experiment

### In-vivo assay I

Evaluation assay of LMP7 inhibitors in a subcutaneous xenograft model of multiple myeloma cells MM. 1S in mice

### 1. Experiment materials

### 1.1 Experimental instrument

Digital Vernier Caliper (Model: 36-111-23, resolution: 0.01 Mm, STANLEY. Stanley), JEB2002 electronic balance (resolution: 0.01 g, Shanghai Puchun Metrology Instrument Co. Ltd.), XS105 electronic balance (resolution: 0.01 mg, Mettler-Toledo Instruments Ltd.), AL204 electronic balance (resolution: 0.1 Mg, Mettler-Toledo Instrument Co. Ltd.), Haier biosafety cabinet (model: HR40-II A2, Qingdao Haier Special Electric Co. Ltd.).

### 1.2 Experimental animals

Female NOD/SCID mice, week old: 5-6 weeks old, certificate No: SCXK(Jing) 2021-0006, purchased from Beijing Vital River Experimental Animal Technology Co. Ltd.

### 2. Experimental method

### Grouping and administration of mice

The specific method was that mice were housed in an SPF environment. Every 5 mice were housed in 1 cage. Animals were free to drink and eat and were adaptively raised for one week. Laboratory conditions: room temperature 18-24°C, relative humidity 40% -70%, ventilated with a ventilation fan, natural light source for 12 hours per day. Cages, bedding, drinking water, and feed were replaced twice a week, and the replaced cages and drinking water bottles were cleaned and then sterilized by steam under high pressure. Cages, room walls, and floors were cleaned twice a week with 84-disinfectant or bromogeramine.

MM. 1S (Cell Resource Center, Institute of Basic Medical Sciences, Chinese Academy of Medical Sciences) cells were cultured using RPMI-1640 medium supplemented with 20% fetal bovine serum and 1% Penicillin-Streptomycin at 37°C in an atmosphere of 5% CO₂ in air. Cells were routinely sub-cultured twice weekly, and cells grown in the exponential growth phase were harvested and counted. Cell suspensions were mixed well in 30% Matrigel ^{™}(Corning Matrigel^{™} High Concentration Basement Membrane) for tumor cell inoculation.

Mice were pretreated with cyclophosphamide via intraperitoneal injection for 3 days, and each mouse was subcutaneously injected with 0.1 mL of PBS-suspended MM. 1S cell suspension (containing 1.0 × 10⁷ cells, 30% Matrigel) at the axillary sites on both sides to establish a subcutaneous transplanted tumor model of multiple myeloma cells MM. 1S in mice. When the tumor size reached 400-700 mm³, the tumor mass was collected aseptically and cryopreserved for future use. The MM. 1S tumor mass was revived, and the tumor mass was aseptically collected for 2 generations for an *in-vivo* efficacy evaluation assay. When the average tumor volume was about 100-150 mm³, 20 tumor-bearing mice with a relatively regular shape and uniform size were selected for the experiment. They were divided into 4 groups, with 5 animals in each group. The first group of the model group was orally administered with the vehicle once daily; the second group was orally administered with Example 9 (2 mg/kg) once daily; the third group was orally administered with Example 67 (2 mg/kg) once daily; the fourth group was orally administered Example 73 (2 mg/kg) once daily. Administration was started on the day of grouping and continued for 14 days. Tumor diameter and body weight were measured 3 times per week during the experiment. At the end of the experiment, intraocular canthus blood was collected and mice were sacrificed immediately after blood collection to collect tumor tissue.

The study endpoints were tumor growth inhibition, maximum tumor burden (individual tumor size equal to 10% of body weight), and weight loss greater than 20% of body weight at the start of treatment. The tumor volume was calculated as V = 0.5 × a × b², where V is the tumor volume and a and b are the length and width of the tumor, respectively. The tumor growth inhibition rate was calculated as TGI (%) = (1-(tumor volume of treatment group-tumor volume of treatment group at grouping)/(tumor volume of control group-tumor volume of control group at grouping)) * 100%. Complete tumor regression (CR) is defined as a tumor that is reduced to below the palpation limit (mm³). Partial tumor regression (PR) is defined as a tumor that is reduced by at least half compared to the initial tumor volume. The minimum duration of CR or PR in three or more consecutive tumor measurements is required to consider CR or PR as persistent.

Summary statistics (including mean and standard error of the mean (SEM)) are provided for tumor volumes in each group at each time point and the differences in tumor volumes are shown in the corresponding study tables. T-test can be used to compare the significance between groups, and a p-value<0.05 indicates a statistical difference.

### 3. Experimental results

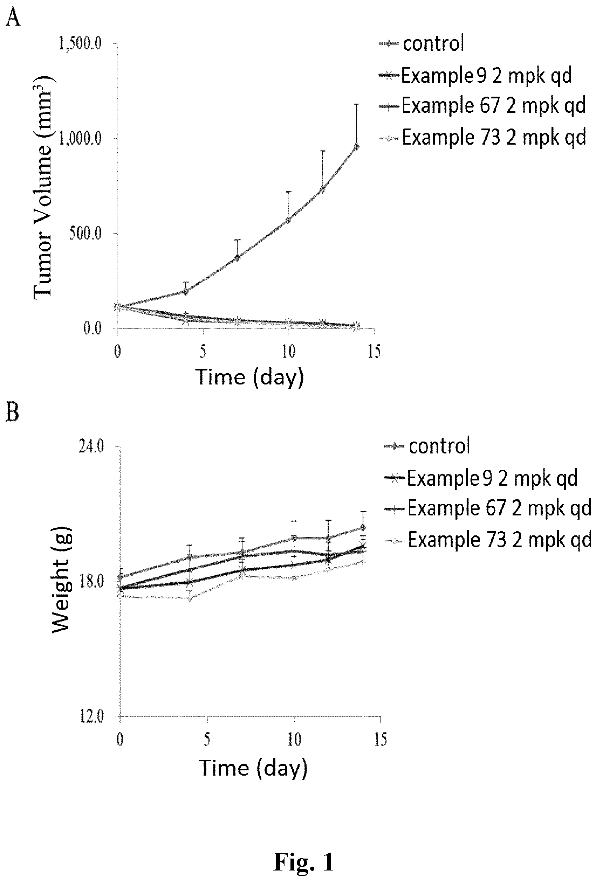

The results are shown in FIG. 1 (A is the tumor growth curve, B is the tumor-bearing mice body weight curve)

It can be seen from FIG. 1 that at the end of the experiment (Day 14), vehicle control (Vehicle) mice had tumor volumes up to 957.04 mm³. TGI and T/C of Example 9 (2 mg/kg) were 112.28% and 0.88%, respectively, demonstrating significant tumor inhibition (p <0.01); (2 mg/kg) TGI and T/C of example 67 were 112.06% and 1.57%, respectively, demonstrating significant tumor inhibition (*p* <0.01); TGI and T/C of example 73 (2 mg/kg) were 112.56% and 0.53%, respectively, demonstrating significant tumor inhibition (*p* <0.001). At the same time, no animal death or weight loss caused by the compound was observed during the experiment, indicating good safety of the compound. The results show that example 9, example 67, and example 73 hold good promise as oral small-molecule LMP7 inhibitors for the treatment of multiple myeloma.

### In-vivo assay II

Evaluation assay of LMP7 Inhibitors in a subcutaneous xenograft model of multiple myeloma cells U266B1 in mice

### 1. Experiment materials

### 1.1 Experimental instrument

Digital Vernier Caliper (Model: 36-111-23, resolution: 0.01 Mm, STANLEY. Stanley), JEB2002 electronic balance (resolution: 0.01 g, Shanghai Puchun Metrology Instrument Co. Ltd.), XS105 electronic balance (resolution: 0.01 mg, Mettler-Toledo Instruments Ltd.), AL204 electronic balance (resolution: 0.1 Mg, Mettler-Toledo Instrument Co. Ltd.), Haier biosafety cabinet (model: HR40-II A2, Qingdao Haier Special Electric Co. Ltd.).

### 1.2 Experimental animals

Female NOG mice, week old: 6-8 weeks old, certificate No: SCXK(Jing) 2021-0006, purchased from Beijing Vital River Experimental Animal Technology Co. Ltd.

### 2. Experimental method

### Grouping and administration of mice

The specific method was that mice were housed in an SPF environment. Every 5 mice were housed in 1 cage. Animals were free to drink and eat and were adaptively raised for one week. Laboratory conditions: room temperature 18-24°C, relative humidity 40% -70%, ventilated with a ventilation fan, natural light source for 12 hours per day. Cages, bedding, drinking water, and feed were replaced twice a week, and the replaced cages and drinking water bottles were cleaned and then sterilized by steam under high pressure. Cages, room walls, and floors were cleaned twice a week with 84-disinfectant or bromogeramine.

U266b1 (China Center for Type Culture Collection, College of Life Sciences at Wuhan University) cells were cultured using RPMI-1640 medium supplemented with 20% fetal bovine serum and 1% Penicillin-Streptomycin at 37°C in an atmosphere of 5% CO₂ in the air. Cells were routinely sub-cultured twice weekly, and cells grown in the exponential growth phase were harvested and counted. Cell suspensions were mixed well at 1 : 1 in Matrigel ^{™}(Corning Matrigel^{™} Normal Basement Membrane) for tumor cell inoculation.

Each mouse was subcutaneously injected with 0.1 ml of PBS-suspended U266B1 cell suspension (containing 1.0 × 10⁷ cells, 50% Matrigel) at the axillary sites on both sides to establish a subcutaneous transplanted tumor model of multiple myeloma cells U266B1 in mice. When the average tumor volume was about 100-150 mm³, 10 tumor-bearing mice with a relatively regular shape and uniform size were selected for the experiment. They were divided into 2 groups, with 5 animals in each group. The first group of the model group was orally administered with vehicle once daily; the second group was orally administered with example 9 (0.3 mg/kg) once daily. Administration was started on the day of grouping and continued for 14 days. Tumor diameter and body weight were measured 3 times per week during the experiment. At the end of the experiment, intraocular canthus blood was collected and mice were sacrificed immediately after blood collection to collect tumor tissue.

The study endpoints were tumor growth inhibition, maximum tumor burden (individual tumor size equal to 10% of body weight), and weight loss greater than 20% of body weight at the start of treatment. The tumor volume was calculated as V = 0.5 × a × b², where V is the tumor volume and a and b are the length and width of the tumor, respectively. The tumor growth inhibition rate was calculated as TGI (%) = (1-(tumor volume of treatment group-tumor volume of treatment group at grouping)/(tumor volume of control group-tumor volume of control group at grouping)) * 100%. Complete tumor regression (CR) is defined as a tumor that is reduced to below the palpation limit (mm³). Partial tumor regression (PR) is defined as a tumor that is reduced by at least half compared to the initial tumor volume. The minimum duration of CR or PR in three or more consecutive tumor measurements is required to consider CR or PR as persistent.

Summary statistics (including mean and standard error of the mean (SEM)) are provided for tumor volumes in each group at each time point and the differences in tumor volumes are shown in the corresponding study tables. T-test can be used to compare the significance between groups, and a p-value<0.05 indicates a statistical difference.

### 4. Experimental results

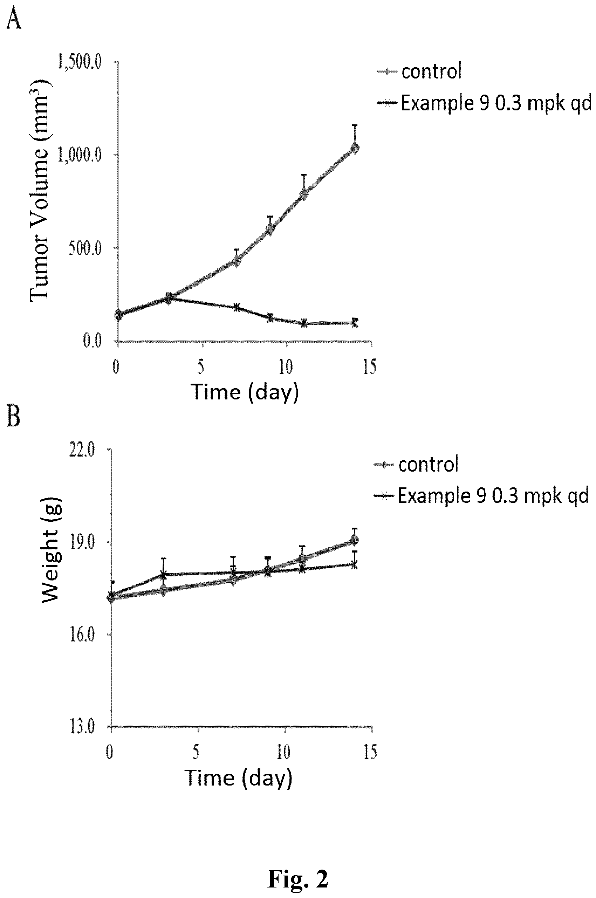

The results are shown in FIG. 2 (A is the tumor growth curve, B is the tumor-bearing mice body weight curve)

It can be seen from FIG. 1 that at the end of the experiment (Day 14), vehicle control (Vehicle) mice had tumor volumes up to 1041.51 mm³. TGI and T/C of example 9 (0.3 mg/kg) were 104.45% and 9.76%, respectively, demonstrating significant tumor inhibition (p <0.001). At the same time, no animal death or weight loss caused by the compound was observed during the experiment, indicating good safety of the compound. The results show that example 9 holds good promise as an oral small molecule LMP7 inhibitor for the treatment of multiple myeloma.

In summary, the results show that LMP7 inhibitors (example 9, example 67, example 73) have a good tumor inhibition effect on multiple myeloma.

## Claims

1. A compound of formula (I), or a pharmaceutically acceptable salt, solvate, polymorph, or tautomer thereof, 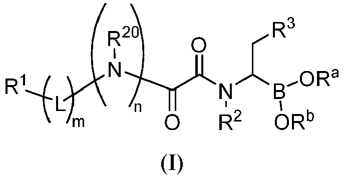 wherein,
R^{a} and R^{b} are each independently selected from H and C₁₋₆ alkyl, or R^{a} and R^{b} can join together to form a 3-10 membered heterocycle, wherein the heterocycle can be optionally substituted with (C=O), halogen, or C₁₋₆ alkyl; the alkyl can be optionally substituted with -COOH; the heterocycle can be optionally fused with a phenyl ring or a 5-6 membered heteroaryl ring; the phenyl ring or 5-6 membered heteroaryl ring can be optionally substituted with halogen or C₁₋₆ alkyl,
R² and R²⁰ are each independently selected from H and C₁₋₆ alkyl,
L is -CR⁴R⁵-,
R⁴ and R⁵ are each independently selected from H, C₁₋₆ alkyl and 3-8 membered cycloalkyl,
R¹ is selected from -OH, C₁₋₆ alkyl, -O-C₁₋₆ alkyl, C₂₋₆ alkenyl, C₂₋₆ alkynyl, 3-12 membered cycloalkyl, 3-12 membered heterocycloalkyl, 6-10 membered aryl, and 5-12 membered heteroaryl, wherein the alkyl, cycloalkyl and heterocycloalkyl can be optionally substituted with (=O); the cycloalkyl, heterocycloalkyl, aryl and heteroaryl can be optionally substituted with halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CHz)₀₋₃-NH-(CO)-R⁶, 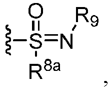 or 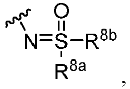 or,
R¹ and R²⁰ join together to form a 3-12 membered heterocycle, wherein the heterocycle can be optionally substituted with (=O), halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 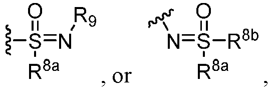
R³ is selected from C₁₋₆ alkyl, 6-10 membered aryl, and 5-12 membered heteroaryl, wherein the aryl and heteroaryl can be optionally substituted with halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, or -(CH₂)₀₋₃-NH-(CO)-R⁶,
R⁶ are each independently selected from C₁₋₆ alkyl, 3-8 membered cycloalkyl, 3-8 membered heterocycloalkyl, 6-10 membered aryl, and 5-12 membered heteroaryl, wherein the alkyl, cycloalkyl, heterocycloalkyl, aryl and heteroaryl can be optionally substituted with halogen, -CN, C₁₋₆ alkyl, -O-C₁₋₆ alkyl, 3-8 membered cycloalkyl, 3-8 membered heterocycloalkyl, 6-10 membered aryl, or 5-12 membered heteroaryl,
R¹⁰ are each independently selected from H and C₁₋₆ alkyl,
R⁷ are each independently selected from 3-8 membered cycloalkyl, 3-8 membered heterocycloalkyl, 6-10 membered aryl, and 5-12 membered heteroaryl, wherein the cycloalkyl, heterocycloalkyl, aryl and heteroaryl can be optionally substituted with halogen, -CN, -OH, -NH₂, NO₂, -O-C₁₋₆ alkyl, -N(C₁₋₆ alkyl) (C₁₋₆ alkyl), or C₁₋₆ alkyl,
R^{8a} and R^{8b} are each independently selected from C₁₋₆ alkyl, 3-8 membered cycloalkyl, 3-8 membered heterocycloalkyl, 6-10 membered aryl, and 5-12 membered heteroaryl, or
R^{8a} and R^{8b} can join together to form a 3-8 membered heterocycle,
R⁹ is selected from H, C₁₋₆ alkyl, 3-8 membered cycloalkyl, 3-8 membered heterocycloalkyl, 6-10 membered aryl, and 5-12 membered heteroaryl,
m is 0, 1, 2 or 3,
n is 0 or 1.

2. The compound according to claim 1, or a pharmaceutically acceptable salt, solvate, polymorph, or tautomer thereof, wherein
R^{a} and R^{b} are each independently selected from H and C₁₋₆ alkyl, or R^{a} and R^{b} can join together to form a 3-10 membered heterocycle,
R¹ is selected from C₁₋₆ alkyl, -O-C₁₋₆ alkyl, C₂₋₆ alkenyl, C₂₋₆ alkynyl, 3-12 membered cycloalkyl, 3-12 membered heterocycloalkyl, 6-10 membered aryl, and 5-12 membered heteroaryl, wherein the alkyl, cycloalkyl and heterocycloalkyl can be optionally substituted with (=O); the cycloalkyl, heterocycloalkyl, aryl and heteroaryl can be optionally substituted with halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 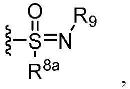 or 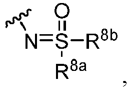 or,
R¹ and R²⁰ join together to form a 3-12 membered heterocycle, wherein the heterocycle can be optionally substituted with (=O), halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 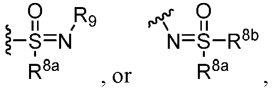
R⁶, R⁷, R^{8a}, R^{8b}, R⁹ and R¹⁰ are as defined in claim 1.

3. The compound according to claim 1, or a pharmaceutically acceptable salt, solvate, polymorph, or tautomer thereof, wherein R² is H.

4. The compound according to claim 1, or a pharmaceutically acceptable salt, solvate, polymorph, or tautomer thereof, wherein R⁴ and R⁵ are each independently selected from H and C₁₋₆ alkyl.

5. The compound according to claim 1, or a pharmaceutically acceptable salt, solvate, polymorph, or tautomer thereof, wherein R³ is selected from 5-12 membered heteroaryl, and the heteroaryl can be optionally substituted with halogen, -CN, -OH, -NH₂, -CF₃, -O-C₁₋₆ alkyl, -NHC₁₋₆ alkyl, C₁₋₆ alkyl, 3-8 membered cycloalkyl, 3-8 membered heterocycloalkyl, 6-10 membered aryl, or 5-12 membered heteroaryl .

6. The compound according to claim 1, or a pharmaceutically acceptable salt, solvate, polymorph, or tautomer thereof, wherein R³ is 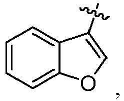 and the 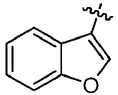 can be optionally substituted with halogen, -CN, -OH, -NH₂, -CF₃, -O-C₁₋₆ alkyl, -NHC₁₋₆ alkyl, C₁₋₆ alkyl, 3-8 membered cycloalkyl, 3-8 membered heterocycloalkyl, 6-10 membered aryl, or 5-12 membered heteroaryl .

7. The compound according to claim 1, or a pharmaceutically acceptable salt, solvate, polymorph, or tautomer thereof, wherein R¹ is selected from 3-8 membered cycloalkyl, 3-8 membered heterocycloalkyl, 6-10 membered aryl, and 5-12 membered heteroaryl, wherein the cycloalkyl and heterocycloalkyl can be optionally substituted with (=O); the cycloalkyl, heterocycloalkyl, aryl and heteroaryl can be optionally substituted with halogen, -CN, -OH, -NH₂, NO₂, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 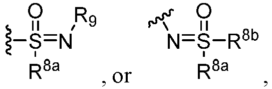 or,
R¹ and R²⁰ join together to form a 3-12 membered heterocycle, wherein the heterocycle can be optionally substituted with (=O), halogen, -CN, -OH, -NH₂, NOz, -(CH₂)₀₋₆-R⁷, -(CH₂)₀₋₆-CF₃, -O-R⁶, -NR¹⁰R⁶, C₁₋₆ alkyl, -(CH₂)₀₋₃-(CO)-R⁶, -(CH₂)₀₋₃-(CO)-NH-R⁶, -(CH₂)₀₋₃-NH-(CO)-R⁶, 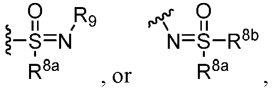
R⁶ are each independently selected from C₁₋₆ alkyl and 3-8 membered cycloalkyl,
R¹⁰ are each independently selected from Hand C₁₋₆ alkyl,
R⁷ are each independently selected from 3-8 membered cycloalkyl and 3-8 membered heterocycloalkyl, wherein the cycloalkyl and heterocycloalkyl can be optionally substituted with halogen, -CN, -OH, -NH₂, NOz, -O-C₁₋₆ alkyl, -N(C₁₋₆ alkyl)(C₁₋₆ alkyl), or C₁₋₆ alkyl,
R^{8a} and R^{8b} are each independently selected from C₁₋₆ alkyl and 3-8 membered cycloalkyl, or
R^{8a} and R^{8b} can join together to form a 3-8 membered heterocycle,
R⁹ is selected from H, C₁₋₆ alkyl and 3-8 membered cycloalkyl.

8. The compound according to claim 1, or a pharmaceutically acceptable salt, solvate, polymorph, or tautomer thereof, wherein m is 0 or 1, n is 1.

9. The compound below 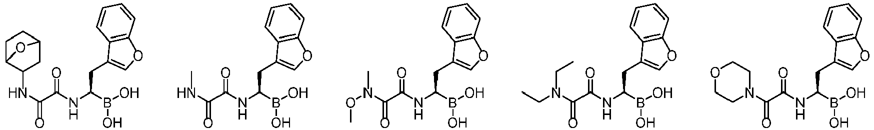 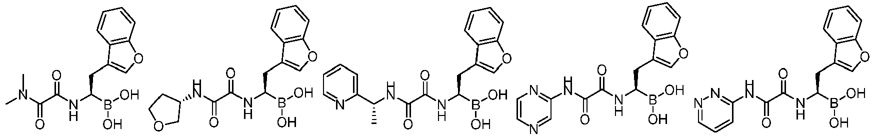 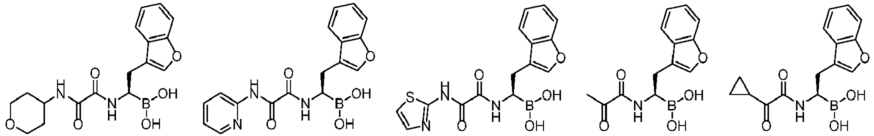 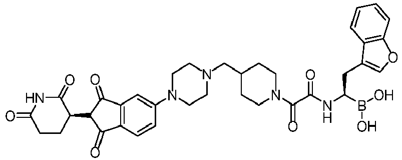 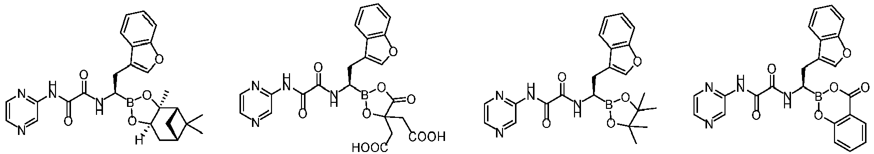 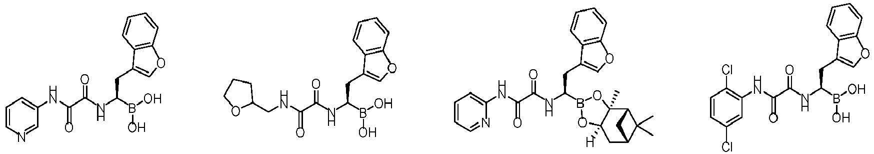 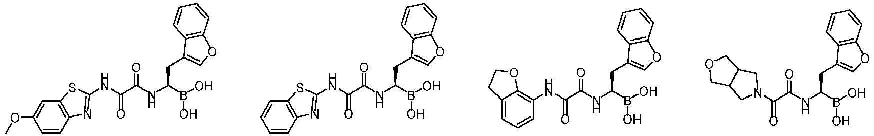 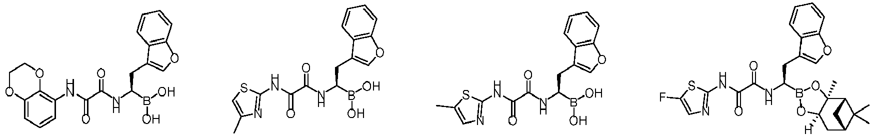 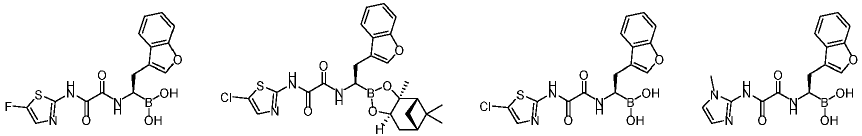 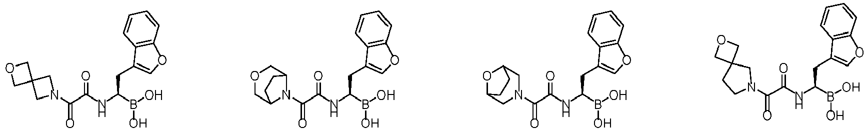 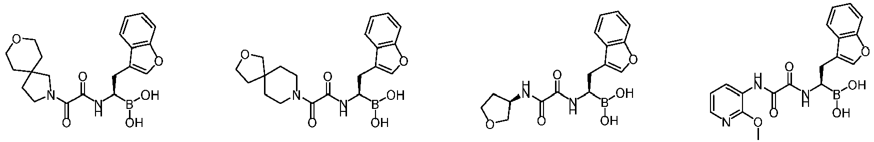 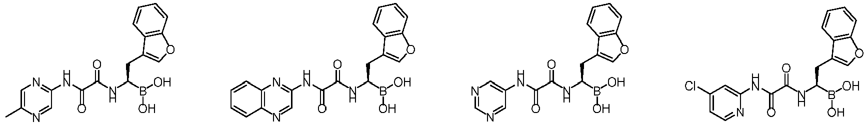 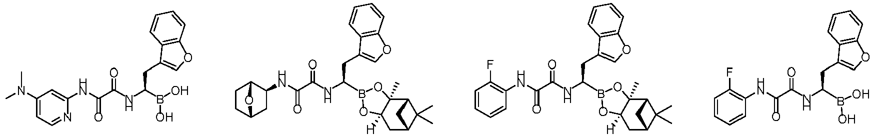 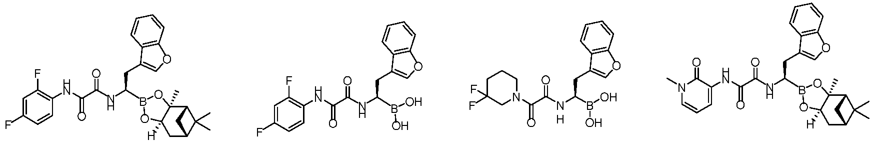 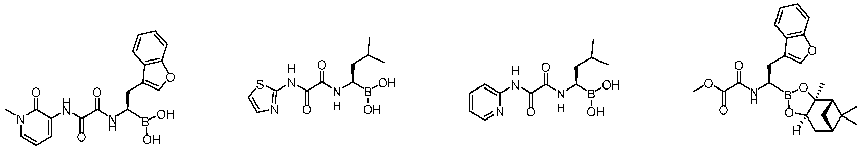 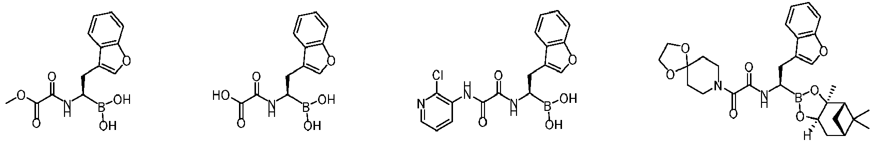 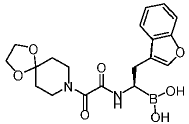 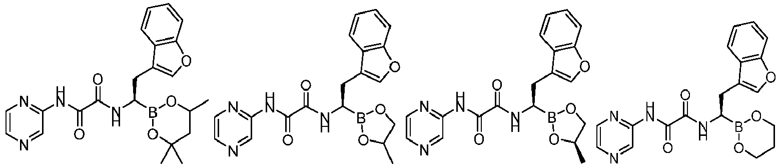 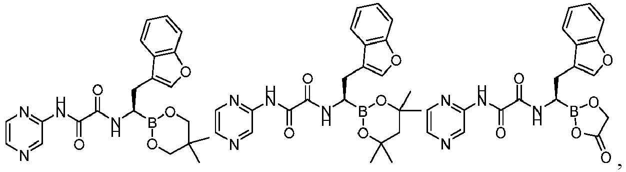 or a pharmaceutically acceptable salt, solvate, polymorph, or tautomer thereof.

10. A pharmaceutical composition comprising the compound according to any one of claims 1-9, or a pharmaceutically acceptable salt, polymorph, or isomer thereof, and a pharmaceutically acceptable excipient.

11. Use of the compound according to any one of claims 1-9, or the pharmaceutically acceptable salt, solvate, polymorph, or isomer thereof or the composition according to claim 10, in the manufacture of a medicament for the treatment of lmp7 activity-related diseases.

12. The use according to claim 11, wherein the lmp7 activity-related disease is multiple myeloma, acute myeloid leukemia, myeloid leukemia, mantle cell lymphoma, chronic lymphocytic leukemia, acute lymphocytic leukemia, diffuse large B-cell lymphoma, plasmacytoma, follicular lymphoma, immunocytoma, breast cancer, liver cancer, colorectal cancer, ovarian cancer, esophageal cancer, lung cancer, head and neck cancer, pancreatic cancer, renal cancer, gastric cancer, thyroid cancer, prostate cancer, bladder cancer, rheumatoid arthritis, systemic lupus erythematosus, inflammatory bowel disease, multiple sclerosis, scleroderma, ankylosing spondylitis, atherosclerosis, Behcet's disease, Crohn's disease, inflammatory bowel disease, ulcerative colitis, autoimmune hepatitis, Sjogren's syndrome, lupus nephritis, asthma, amyotrophic lateral sclerosis (ALS), psoriasis, immunoglobulin A nephropathy, allergic purpura, and Alzheimer's disease (AD).
